# EUROPÄISCHE PATENTSCHRIFT

(11) **EP 1 539 967 B1**
(45) Veröffentlichungstag und Bekanntmachung des Hinweises auf die Patenterteilung: **01.04.2009**
(21) Anmeldenummer: 03753387.4
(22) Anmeldetag: 05.09.2003
(51) Int. Cl.: C12N 15/82, A01H 5/00

(54) **EXPRESSIONSKASSETTEN ZUR EXPRESSION VON NUKLEINSÄUREN IN KOHLENHYDRAT-SPEICHERNDEN SINK-GEWEBEN VON PFLANZEN**
EXPRESSION CASSETTES FOR EXPRESSING NUCLEIC ACID SEQUENCES IN SINK TISSUES OF PLANTS THAT STORE CARBOHYDRATE
CASSETTES D'EXPRESSION DESTINEES A L'EXPRESSION D'ACIDES NUCLEIQUES DANS DES TISSUS DE PUITS DE PLANTES STOCKANT DES GLUCIDES

(30) Priorität: 10.09.2002 DE 10242204
(43) Veröffentlichungstag der Anmeldung: 15.06.2005
(73) Patentinhaber: Sungene GmbH & Co. KGaA, 06466 Gatersleben (DE)
(72) Erfinder: HEIM, Ute, 06466 Gatersleben (DE); HERBERS, Karin, 06484 Quedlinburg (DE); SONNEWALD, Uwe, 06484 Quedlinburg (DE)
(74) Vertreter: Bieberbach, Andreas
(86) Internationale Anmeldenummer: PCT/EP2003/009855
(87) Internationale Veröffentlichungsnummer: WO 2004/024926

(56) Entgegenhaltungen:
- WO-A-00/26388
- WO-A-98/40503
- BUCHNER P ET AL: "GLUCAN PHOSPHORYLASES IN VICIA FABA L.: CLONING, STRUCTURAL ANALYSIS AND EXPRESSION PATTERNS OF CYTOSOLIC AND PLASTIDIC FORMS IN RELATION TO STARCH" PLANTA, SPRINGER VERLAG, DE, Bd. 199, 1996, Seiten 64-73, XP002071466 ISSN: 0032-0935 in der Anmeldung erwähnt
- BRISSON N ET AL: "MATURATION AND SUBCELLULAR COMPARTMENTATION OF POTATO STARCH PHOSPHORYLASE" PLANT CELL, AMERICAN SOCIETY OF PLANT PHYSIOLOGISTS, ROCKVILLE, MD, US, Bd. 1, Nr. 5, 1. Mai 1989 (1989-05-01), Seiten 559-566, XP000095141 ISSN: 1040-4651
- ST-PIERRE BENOIT ET AL: "5' deletion analysis of the potato starch phosphorylase gene: An upstream sequence defines distal regulatory elements and a proximal organ-dependent promoter" PLANT SCIENCE (LIMERICK), Bd. 110, Nr. 2, 1995, Seiten 193-203, XP002270538 ISSN: 0168-9452 in der Anmeldung erwähnt
- ST-PIERRE BENOIT ET AL: "The starch phosphorylase gene is subjected to different modes of regulation in starch-containing tissues of potato" PLANT MOLECULAR BIOLOGY, Bd. 30, Nr. 6, 1996, Seiten 1087-1098, XP002270539 ISSN: 0167-4412 in der Anmeldung erwähnt

### SEQUENZPROTOKOLL

<110> SunGene GmbH & Co.KGaA
<120> Transgene Expressionskassetten zur Expression von Nukleinsäuren in Kohlenhydrat-speichernden Sink-Geweben von Pflanzen
<130> AE20020414
<140>
   <141>
<160> 8
<170> PatentIn Ver. 2.1
<210> 1
   <211> 1300
   <212> DNA
   <213> Vicia faba
<220>
   <221> promoter
   <222> (1)..(1300)
<400> 1
   gattgtctct agatgtaggt gtggttttat cgaactgagt taacagactt tgtgtgttct 60
   ttatttcttt tgtcttgaat tattgttact gtcatattgt gtgttatttg atttaacaag 120
   taaaactatc tgatttaaca agtaaagcaa gttattatag acatcttttc caacatctgt 180
   cccgtgagaa acaaaattat aacttggaag acttgaatta aggtggtgtc ttgaaaaaat 240
   aatatacttc gtcagtgcaa catgttgcag taaatgtgca acaggttaca ccccttcatt 300
   gcaatgttca atttcagaca gagatttggc acaataagtt gcagtaaatg tgtaacatgt 360
   tgcaccattc cagctttata ctttttgaga caaagaatta gtgcaatcaa ttataggtat 420
   gagaaactta tttttctcat tagttggtca gctgtactat ttgtatgata gtttaaatat 480
   caagttggat catttatgtt ataatgccat ctttccttcc taattctctc tatttctctc 540
   attatctcaa tctttctatt attcactatt gttttcgtga ttgaaatttt aatccaacaa 600
   ataaatgaaa acgcaacaca attgataaaa tacatcaaac ttaagttaaa ggtgtaagtt 660
   tgagtcttga tttagtataa aattttatct atcaacatta taatttatta attttaaatt 720
   ttttattatt tatatattta caaatacaat ttctgattta aatataataa ataacattaa 780
   aaatatattt ttaaataaca ttatataaat tatgtgacgt aacaccaaat gattattgtc 840
   atatcaatat cacgccataa attatgggat caactatata aatttgaaga ggataaaaag 900
   aaggaaaaaa aatattaaat ataggaaaaa actgtttttt taaaaggact aaagttttgt 960
   atatcaaaat caaaatcaaa attgaaatta aaatttcatt tttatattaa aaacaaaaaa 1020
   aaagcttgag tttaatttta aacacaagtc aatattcttc tccaagtgta aaaactcaca 1080
   atcggcgctt tttaaaggaa aaaatgactt ttcacgaccc gcgtttggag gtgctgtaac 1140
   gtggcaataa gtcacgaaag agcgtgcgtg gtagtacaaa aaaaactaaa atccagaagc 1200
   taaaagctcg tgattcgatg ccacatcata ttctttttca acactagtaa gagtacagtg 1260
   tagaatacaa aacaccacct ctattataga gagaagaggt                      1300
<210> 2
   <211> 1360
   <212> DNA
   <213> Vicia faba
<220>
   <221> promoter
   <222> (1)..(1300)
<220>
   <221> 5'UTR
   <222> (1301)..(1357)
<220>
   <221> misc_feature
   <222> (1358)..(1360)
   <223> ATG-Start-Codon
<400> 2
   gattgtctct agatgtaggt gtggttttat cgaactgagt taacagactt tgtgtgttct 60
   ttatttcttt tgtcttgaat tattgttact gtcatattgt gtgttatttg atttaacaag 120
   taaaactatc tgatttaaca agtaaagcaa gttattatag acatcttttc caacatctgt 180
   cccgtgagaa acaaaattat aacttggaag acttgaatta aggtggtgtc ttgaaaaaat 240
   aatatacttc gtcagtgcaa catgttgcag taaatgtgca acaggttaca ccccttcatt 300
   gcaatgttca atttcagaca gagatttggc acaataagtt gcagtaaatg tgtaacatgt 360
   tgcaccattc cagctttata ctttttgaga caaagaatta gtgcaatcaa ttataggtat 420
   gagaaactta tttttctcat tagttggtca gctgtactat ttgtatgata gtttaaatat 480
   caagttggat catttatgtt ataatgccat ctttccttcc taattctctc tatttctctc 540
   attatctcaa tctttctatt attcactatt gttttcgtga ttgaaatttt aatccaacaa 600
   ataaatgaaa acgcaacaca attgataaaa tacatcaaac ttaagttaaa ggtgtaagtt 660
   tgagtcttga tttagtataa aattttatct atcaacatta taatttatta attttaaatt 720
   ttttattatt tatatattta caaatacaat ttctgattta aatataataa ataacattaa 780
   aaatatattt ttaaataaca ttatataaat tatgtgacgt aacaccaaat gattattgtc 840
   atatcaatat cacgccataa attatgggat caactatata aatttgaaga ggataaaaag 900
   aaggaaaaaa aatattaaat ataggaaaaa actgtttttt taaaaggact aaagttttgt 960
   atatcaaaat caaaatcaaa attgaaatta aaatttcatt tttatattaa aaacaaaaaa 1020
   aaagcttgag tttaatttta aacacaagtc aatattcttc tccaagtgta aaaactcaca 1080
   atcggcgctt tttaaaggaa aaaatgactt ttcacgaccc gcgtttggag gtgctgtaac 1140
   gtggcaataa gtcacgaaag agcgtgcgtg gtagtacaaa aaaaactaaa atccagaagc 1200
   taaaagctcg tgattcgatg ccacatcata ttctttttca acactagtaa gagtacagtg 1260
   tagaatacaa aacaccacct ctattataga gagaagaggt acaatacaaa caatcaaagc 1320
   tctgtgagtg tgtgagtgag tgagagaaat tccaaccatg                       1360
<210> 3
   <211> 1582
   <212> DNA
   <213> Vicia faba
<220>
   <221> promoter
   <222> (1)..(1300)
<220>
   <221> 5'UTR
   <222> (1301)..(1357)
<220>
   <221> transit_peptide
   <222> (1358)..(1582)
<400> 3
   gattgtctct agatgtaggt gtggttttat cgaactgagt taacagactt tgtgtgttct 60
   ttatttcttt tgtcttgaat tattgttact gtcatattgt gtgttatttg atttaacaag 120
   taaaactatc tgatttaaca agtaaagcaa gttattatag acatcttttc caacatctgt 180
   cccgtgagaa acaaaattat aacttggaag acttgaatta aggtggtgtc ttgaaaaaat 240
   aatatacttc gtcagtgcaa catgttgcag taaatgtgca acaggttaca ccccttcatt 300
   gcaatgttca atttcagaca gagatttggc acaataagtt gcagtaaatg tgtaacatgt 360
   tgcaccattc cagctttata ctttttgaga caaagaatta gtgcaatcaa ttataggtat 420
   gagaaactta tttttctcat tagttggtca gctgtactat ttgtatgata gtttaaatat 480
   caagttggat catttatgtt ataatgccat ctttccttcc taattctctc tatttctctc 540
   attatctcaa tctttctatt attcactatt gttttcgtga ttgaaatttt aatccaacaa 600
   ataaatgaaa acgcaacaca attgataaaa tacatcaaac ttaagttaaa ggtgtaagtt 660
   tgagtcttga tttagtataa aattttatct atcaacatta taatttatta attttaaatt 720
   ttttattatt tatatattta caaatacaat ttctgattta aatataataa ataacattga 780
   aaatatattt ttaaataaca ttatataaat tatgtgacgt aacaccaaat gattattgtc 840
   atatcaatat cacgccataa attatgggat caactatata aatttgaaga ggataaaaag 900
   aaggaaaaaa aatattaaat ataggaaaaa actgtttttt taaaaggact aaagttttgt 960
   atatcaaaat caaaatcaaa attgaaatta aaatttcatt tttatattaa aaacaaaaaa 1020
   aaagcttgag tttaatttta aacacaagtc aatattcttc tccaagtgta aaaactcaca 1080
   atcggcgctt tttaaaggaa aaaatgactt ttcacgaccc gcgtttggag gtgctgtaac 1140
   gtggcaataa gtcacgaaag agcgtgcgtg gtagtacaaa aaaaactaaa atccagaagc 1200
   taaaagctcg tgattcgatg ccacatcata ttctttttca acactagtaa gagtacagtg 1260
   tagaatacaa aacaccacct ctattataga gagaagaggt acaatacaaa caatcaaagc 1320
   tctgtgagtg tgtgagtgag tgagagaaat tccaattatg gcttccatga caatgcggtt 1380
   tcatccaaat tccaccgccg taaccgaatc cgttcctcgc cgtggctccg tttacggatt 1440
   catcggttac agatcctcgt cgttgttcgt ccgaacgaac gttatcaagt atcgttctgt 1500
   taagcgtaat ctggaattta ggaggagaag cgctttctct gtgaagtgtg gttctggtaa 1560
   tgaagcgaaa cagaaagcca tg 1582
<210> 4
   <211> 26
   <212> DNA
   <213> Künstliche Sequenz
<220>
   <223> Beschreibung der künstlichen Sequenz: Oligonucleotide primer
<400> 4
   gattgtctct agatgtaggt gtgttt 26
<210> 5
   <211> 24
   <212> DNA
   <213> Künstliche Sequenz
<220>
   <223> Beschreibung der künstlichen Sequenz: Oligonucleotide primer
<400> 5
   catggaagcc atggttgaat ttct 24
<210> 6
   <211> 27
   <212> DNA
   <213> Künstliche Sequenz
<220>
   <223> Beschreibung der künstlichen Sequenz: Oligonucleotide primer
<400> 6
   ttcctgatcc atggctttct gtttcgc 27
<210> 7
   <211> 225
   <212> DNA
   <213> Vicia faba
<220>
   <221> transit_peptide
   <222> (1)..(225)
<220>
   <221> CDS
   <222> (1)..(225)
<400> 7
   atg gct tcc atg aca atg cgg ttt cat cca aat tcc acc gcc gta acc    48
   Met Ala Ser Met Thr Met Arg Phe His Pro Asn Ser Thr Ala Val Thr
   1               5                   10                  15
   gaa tcc gtt cct cgc cgt ggc tcc gtt tac gga ttc atc ggt tac aga    96
   Glu Ser Val Pro Arg Arg Gly Ser Val Tyr Gly Phe Ile Gly Tyr Arg
               20                  25                  30
   tcc tcg tcg ttg ttc gtc cga acg aac gtt atc aag tat cgt tct gtt    144
   Ser Ser Ser Leu Phe Val Arg Thr Asn Val Ile Lys Tyr Arg Ser Val
           35                  40                  45
   aag cgt aat ctg gaa ttt agg agg aga agc gct ttc tct gtg aag tgt    192
   Lys Arg Asn Leu Glu Phe Arg Arg Arg Ser Ala Phe Ser Val Lys Cys
       50                  55                  60
   ggt tct ggt aat gaa gcg aaa cag aaa gcc atg                        225
   Gly Ser Gly Asn Glu Ala Lys Gln Lys Ala Met
   65                  70                  75
<210> 8
   <211> 75
   <212> PRT
   <213> Vicia faba
<400> 8
   Met Ala Ser Met Thr Met Arg Phe His Pro Asn Ser Thr Ala Val Thr
    1               5                  10                  15
   Glu Ser Val Pro Arg Arg Gly Ser Val Tyr Gly Phe Ile Gly Tyr Arg
               20                  25                  30
   Ser Ser Ser Leu Phe Val Arg Thr Asn Val Ile Lys Tyr Arg Ser Val
           35                  40                  45
   Lys Arg Asn Leu Glu Phe Arg Arg Arg Ser Ala Phe Ser Val Lys Cys
       50                  55                  60
   Gly Ser Gly Asn Glu Ala Lys Gln Lys Ala Met
   65                  70                  75

## Beschreibung

Die Erfindung betrifft Verfahren zur gezielten, transgenen Expression von Nukleinsäuresequenzen in den Kohlenhydrat-speichernden Sink-Geweben von Pflanzen unter Verwendung von Expressionskaesetten, die den Promotor der plastidären, 1,4-α-D-Glukanphosphat-α-D-glucosyltransferase aus Vicia faba enthalten. Weiterhin betrifft die Erfindung besagte Expressionskassetten und diese enthaltende Expressionsvektoren und transgene Pflanzen enthaltend ein Kohlenhydrat-speicherndes Sink-Gewebe oder Mikroorganismen, sowie die Verwendung derselben zur Herstellung von Nahrungs-, Futtermitteln, Saatgut, Pharmazeutika oder Feinchemikalien.

Ziel biotechnologischer Arbeiten an Pflanzen ist die Herstellung von Pflanzen mit verbesserten Eigenschaften zum Beispiel zur Steigerung der landwirtschaftlichen Produktivität. Transkriptionelle regulatorische Sequenzen oder Promotoren, welche die Expression von Genen in Pflanzen regulieren, sind essentielle Elemente der Pflanzenbiotechnologie. Verschiedene Promotoren, die erfolgreich für die Expression heterologer Gene in Pflanzen verwendet wurden, sind verfügbar und umfassen sowohl pflanzliche Promotoren (wie z.B. die Promotoren des Hitzeschockproteins hsp80 aus Blumenkohl; US 5,612,472) als auch Promotoren aus anderen nicht-pflanzlichen Quellen, wie beispielsweise Promotoren pflanzlicher Viren (z.B. der 35S Promotor des Blumenkohl Mosaik Virus) oder Pflanzen infizierender Bakterien (z.B. der Promotor der Octopin-Synthase aus Agrobakterium; Leisner and Gelvin (1988) Proc Natl Acad Sci USA 85(8):2553-2557).

Für die Expression von heterologen Nukleinsäuresequenzen in transgenen Pflanzen werden häufig sogenannte konstitutive Promotoren eingesetzt, welche in der Pflanze weitgehend zu jeder Zeit und in jedem Gewebe die Expression eines Genproduktes regulieren. Eine gezielte Expression von Genen in bestimmten Pflanzenteilen oder zu bestimmten Entwicklungszeitpunkten ist mit diesen Promotoren nicht möglich. So wird das transgen zu exprimierende Protein an Orten und zu Zeiten exprimiert, wo es nicht erforderlich ist, was beispielsweise unnötig Energie verbraucht, metabolische Veränderungen verursacht und sich so nachteilig auf das Wachstum der Pflanze auswirken kann. Auch aus Gründen der Produktzulassung und -akzeptanz ist es wünschenswert, ein transgenes Protein nur dort zu exprimieren, wo es aufgrund seines angestrebten Effektes benötigt wird.

Zu diesem Zweck genießen gewebe- und entwicklungsspezifische Promotoren ein großes Interesse. Verschiedene solche Promotoren sind bekannt. So vermittelt der Promotor des "sucrose bindenden Protein ähnlichen Gens" (SBP) aus *Vicia faba* eine starke und spezifische Expression in Samen von Raps und anderen Pflanzen (WO 00/26388).

Früchte, Samen, Rüben oder Knollen sind als wichtige Speicherorgane pflanzlicher Organismen von hoher agro-ökonomischer Relevanz. Sie dienen der Speicherung von Proteinen, Ölen und Kohlenhydraten (insbesondere Stärke). Derartige Gewebe sind in der Regel photosynthetisch inaktiv und werden auch als Sink-Gewebe oder Sink-Organe bezeichnet. Sie sind auf den Import von Photoassimilaten aus den photosynthetisch aktiven Pflanzenteilen (Source-Organen oder Source-Geweben) angewiesen. Sowohl klassische Züchtung als auch biotechnologische Verfahren wurden für die Verbesserung spezifischer Aspekte der Frucht- und Knollenqualität verwendet. Qualitativ hochwertige, reife Früchte resultieren aus einer Zahl von koordinierten biochemischen und metabolischen Änderungen, welche nicht nur während der Reifung, sondern auch während der Fruchtentwicklung auftreten können. Diese Änderungen bestimmen die Endqualität und die Menge der Früchte. Beispiele für veränderte Eigenschaften beispielsweise bei Tomatenfrüchten sind eine Erhöhung des Saccharoseimportes, Umwandlung in Stärke, Akkumulation von verschiedenen organischen Säuren, Modifikationen von Pigmenten und Änderungen in fungiziden und insektiziden verbindungen. Solche Ergebnisse können durch die Überexpression von Genen/Proteinen oder durch Hemmung mittels doppelsträngiger RNA, antisense-RNA bzw. Co-Suppression erreicht werden. Da Sink-Gewebe als Speicherort der wichtigsten pflanzlichen Rohstoffe fungieren, sind Promotoren, die eine selektive Expression in diesen Geweben ermöglichen, von besonderem Interesse für die Pflanzenbiotechnologie, da sie eine gezielte Modifikation dieser Gewebe und ihrer Inhaltstoffe erlauben.

Verschiedene Promotoren, welche für die Expression von Nukleinsäuresequenzen in Früchten, Samen oder Knollen verwendet werden können, sind dem Fachmann bekannt. Zu nennen ist der Promotor des genomischen Klons 2A11 aus Tomate (Pear et. al. (1989) Plant Mol Biol 13:639-651; WO 91/19806). Der 2A11 Promotor kontrolliert jedoch die Expression in den sehr frühen Stadien und ist relativ schwach. Die Ethylen induzierbaren E4 und E8 Promotoren aus Tomate (US 5,859,330; Deickmann et al. (1988) EMBO J 7:3315-3320) sowie der Promotor der Polygalacturonase (US 6,127,179) sind ebenfalls als fruchtspezifisch beschrieben. Die genannten Promotoren zeigen aber eine Expression nur in den späten Phasen der Fruchtentwicklung und können daher nur limitiert eingesetzt werden. Die Promotoren TFM7 und TFM9 (US 5,608,150) sind während der Fruchtentwicklung in grünen und gelben Stadien aktiv. Die fruchtspezifische Regulation des Actinidin Promotors aus Kiwi konnte für die Expression in Petunien nachgewiesen werden (Lin et al. (1993) Plant Mol Biol 23:489-499). Thi-1, MADS2 und eine Promotorfusion zwischen Thi-1 und dem Actin Promotor der Melone regulieren die Expression heterologer Gene spezifisch in Äpfeln (WO 00/66610).

Weitere Promotoren sind beispielsweise Promotoren mit einer Spezifität für Knollen, Speicherwurzeln oder Wurzeln, wie beispielsweise der knollenspezifische Patatin Promotor Klasse I (Bevan et al. (1986) Nucl Acids Res 14:4625-4638), der Promotor des Cathepsin D Inhibitors aus Kartoffel, der Promotor der Stärke Synthase (GBSS1) oder der Sporamin Promotor. Andere Gene mit spezifischer hoher Aktivität in Knollen sind beispielsweise der Promotor des Gens der ADP Glukosepyrophosphorylase (Müller et al. (1990) Mol Gen Genet 224:136-146), der Sucrosesynthase (Salanoubat und Belliard (1987) Gene 60:47-56; Salanoubat und Belliard (1989) Gene 84:181-185), die Promotoren des 22 kD Protein Komplexes und des Proteinaseinhibitors (Hannapel (1990) Plant Physiol 94:919-925) und der anderen Klasse I Patatine (B33) (EP-A1 0 375 092; Rocha-Sosa et al. (1989) EMBO J 8:23-29). Ein Nachteil des Patatin 1 Promoters besteht darin, dass er durch hohe Saccharose Konzentrationen auch in anderen Geweben als der Knolle induziert wird (Jefferson, R. et al (1990) Plant Mol Biol 14:995-1006).

In allen Organismen, die Stärke oder Glykogen speichern können, werden Glukanphosphorylasen (systematischer Name: 1,4-α-D-Glukanphosphat-α-D-glucosyltransferase; oft auch Stärkephosphorylase; EC 2.4.1.1) gefunden. Das Enzym spaltet terminale α-1,4-verknüpfte Glukosereste unter Bildung von Glukose-1-phosphat aus Glukan-ähnlichen Molekülen ab. In pflanzlichen Zellen existieren zwei Enzyme, die sich in ihrer Lokalisation und Spezifität für Glukane unterscheiden. Die Pho2 Isoform befindet sich im Zytosol und besitzt eine hohe Affinität zu verzweigten Polyglukanen wie löslicher Stärke oder Glykogen. Die Isoform Phol ist im Stroma der Plastiden lokalisiert und bevorzugt unverzweigte Glukanähnliche Amylose und Maltodextrine als Substrat. Von dieser Isoform wurden in Kartoffeln zwei homologe Gene mit einer Identität von 81% gefunden, wobei die erste vorwiegend in Knollen und die zweite in Blättern exprimiert wird (Sonnewald et al. (1995) Plant Mol Biol 27:567-576). Auch aus der Ackerbohne *Vicia faba* wurden zwei Isoformen isoliert (Pho1 und Pho2; Buchner P et. al. (1996) Planta 199: 64-73). Die plastidäre Isoform Pho1 ist vermutlich im Biosyntheseweg der Speicherstärke involviert und ist im wesentlichen in den späten Stadien der Samenentwicklung der Ackerbohne stark exprimiert. Trotz zahlreicher Studien über strukturelle und kinetische Eigenschaften der pflanzlichen Glukanphosphorylasen und ihrer Verteilung in den verschiedenen Geweben ist ihre exakte Rolle im Kohlenhydratmetabolismus unklar. Beschrieben ist der Promotor eines Gens aus aus Kartoffel, das für ein Protein mit 75% Homologie zur Glucanphosphorylase aus Vicia faba kodiert
(St-Pierre B & Brisson N (1995) Plant Science 110:193-203; St-Pierre et al. (1996) Plant Mol Biol 30:1087-1098). Der besagte Promotor weißt neben einer Aktivität in den Knollen eine im Vergleich dazu bis zu 1,5fach höhere Aktivität in den Wurzeln auf. Ebenfalls wurden hohe Aktivitäten in den Blattstielen und im Spross gefunden. Insgesamt war die Aktivität in den Knollen geringer als in Blattstielen, im Sproß, in den Stolonen und in den Wurzeln.

Die im Stand der Technik beschriebenen Promotoren weisen einen oder mehrere der nachfolgenden Nachteile auf:
1) Die Promotoren weisen nicht die gewünschte Expressionsstärke auf und/oder sind nur in wenigen Pflanzenarten aktiv.
2) Die Promotoren sind nur sehr früh oder sehr spät während der Frucht- oder Knollenentwicklung aktiv.
3) Das Expressionsmuster stimmt nicht mit den Erwartungen überein d.h. es zeigen sich beispielsweise unerwünschte Expressionsaktivitäten in weiteren Geweben.
4) Die Expression vieler der genannten Promotoren ist Ethylen abhängig.

Darüberhinaus ist die Zahl der vorhandenen Promotoren stark begrenzt. Insbesondere bei Ansätzen, die eine Expression mehrerer heterologer Nukleinsäuresequenzen erfordern, kann dies zum limitierenden Faktor werden. Die Expression von verschiedenen heterologen Sequenzen in einem pflanzlichen Organismus unter dem gleichen Promotor kann zu einer "Abschaltung" ("epigenic silencing") der entsprechenden Expressionskassetten führen (Mette et al. (1999) EMBO J 18:241-24B).

Es stellte sich daher die Aufgabe, neue Promotoren und von diesen abgeleitete Expressionskassetten bereit zustellen, die eine hohe Spezifität für Sink-Gewebe oder Sink-Organe und eine hohe Aktivität über einen möglichst langen Zeitrahmen der Entwicklung aufweisen. Diese Aufgabe wird durch die vorliegende Erfindung gelöst.

Ein erster Gegenstand der Erfindung betrifft Verfahren zur gezielten, transgenen Expression von Nukleinsäuresequenzen in Kohlenhydrat-speichernden Sink-Geweben von Pflanzen enthaltend ein Kohlenhydrat-speicherndes Sink-Gewebe, wobei nachfolgende Schritte umfasst sind
I. Einbringen einer Expressionskassette in pflanzliche Zellen, wobei die Expressionskassette mindestens nachfolgende Elemente enthält
   a) mindestens eine Promotorsequenz des Gens kodierend für die plastidäre 1,4-α-D-Glukanphosphat-α-D-glucosyltransferase aus Vicia faba, und
   b) mindestens eine weitere Nukleinsäuresequenz.
   wobei mindestens eine der besagten Promotorsequenzen und eine weitere Nukleinsäuresequenz funktionell miteinander verknüpft sind und die weitere Nukleinsäuresequenz in Bezug auf die Promotorsequenz heterolog ist, und
II. Auswahl von transgenen Zellen, die besagte Expressionskassette stabil in das Genom integriert enthalten, und
III. Regeneration von vollständigen Pflanzen aus besagten transgenen Zellen, wobei die Promotersequenz unter ansonsten unveränderten Bedingungen eine Transkription in mindesten einem Kohlenhydrat-speichernden, -synthetisierenden oder - metabolisierenden Sink-Gewebe vermittelt, die höher ist als in einem anderen Gewebe.

Eine Expressionskassette kann gegebenenfalls weitere genetische Kontrollelemente enthalten.
Folglich kann eine der weiteren Nukleinsäuresequenzen in Kohlenhydrat-speichernden Sink-Gewebe jedoch im wesentlichen nicht in Source-Geweben exprimiert werden.

Ein weiterer Gegenstand betrifft Expressionskassetten, wie sie z.B. in dem erfindungsgemäßen Verfahren zum Einsatz kommen können. Bevorzugt umfassen die Expressionskassetten zur gezielten, transgenen Expression von Nukleinsäuresequenzen in den Kohlenhydrat-speichernden Sink-Geweben von Pflanzen,
a) mindestens eine Promotorsequenz des Gens kodierend für die plastidäre 1,4-α-D-Glukanphosphat-α-D-glucosyltransferase aus Vicia faba, und
b) mindestens eine weitere Nukleinsäuresequenz
wobei mindestens eine Promotorsequenz und eine weitere Nukleinsäuresequenz funktionell miteinander verknüpft sind und die weitere Nukleinsäuresequenz in Bezug auf die Promotorsequenz heterolog ist.

Promotorsequenzen, die eine Homologie von mindestens 40% über einen Sequenzabschnitt von mindestens 100 Basenpaaren zu der Sequenz gemäß SEQ ID NO: 1 aufweisen und im wesentlichen die gleiche Promotoraktivität wie die Promotorsequenz gemäß SEQ ID NO: 1 haben, können zur gezielten, transgenen Expression von Nukleinsäuresequenzen in den Kohlenhydrat-speichernden Sink-Geweben von Pflanzen eingesetzt werden.
Fragmente der Promotorsequenz mit einer Länge von mindestens 100 Basenpaaren und im wesentlichen der gleichen Promotoraktivität wie die Promotorsequenz gemäß SEQ ID NO: 1 können ebenfalls zur gezielten, transgenen Expression von Nukleinsäuresequenzen in den Kohlenhydrat-speichernden Sink-Geweben von Pflanzen, eingesetzt werden.

In einer bevorzugten Ausführungsform des erfindungsgemäßen Verfahrens und/oder der erfindungsgemäßen Expressionskassetten meint "Promotorsequenz eines Gens kodierend für die plastidäre 1,4-α-D-Glukanphosphat-α-D-glucosyltransferase aus Vicia faba" Promotorsequenzen, die mindestens eine Sequenz umfassen ausgewählt aus der Gruppe von Sequenzen bestehend aus
i) der Promotorsequenz gemäß SEQ ID NO: 1 und
ii) Promotorsequenzen, die eine Homologie von mindestens 80% über einen Sequenzabschnitt von mindestens 500 Basenpaaren zu der Sequenz gemäß SEQ ID NO: 1 aufweisen und die unter ansonsten unveränderten Bedingungen eine Transkription in mindesten einem Kohlenhydrat-speichernden, - synthetisierenden oder -metabolisierenden Sink-Gewebe vermittelt, die höher ist als in einem anderen Gewebe, und
iii) Fragmenten der Promotorsequenz gemäß i) mit einer Länge von mindestens 500 Basenpaaren und im wesentlichen der gleichen Promotoraktivität die Promotorsequenz gemäß SEQ ID NO: 1 und die die unter ansonsten unveränderten Bedingungen eine Transkription in mindestens einem Kohlenhydrat-speichernden, -synthetisierenden oder -metabolisierenden Sink-Gewebe vermittelt, die höher ist als in einem anderen Gewebe.

Die erfindungsgemäßen Expressionskassetten können weitere genetische Kontrollsequenzen und/oder zusätzliche Funktionselemente enthalten.

Bevorzugt können die Expressionskassetten durch die transgen zu exprimierende Nukleinsäuresequenz die Expression eines von besagter Nukleinsäuresequenz kodierten Proteins, und/oder die Expression eines von besagter Nukleinsäuresequenz kodierter sense-RNA, anti-sense-RNA oder doppelsträngigen RNA ermöglichen.

Ein weiterer Gegenstand der Erfindung betrifft Expressionsvektoren, die eine der erfindungsgemäßen Expressionskassetten enthalten.

Ein weiterer Gegenstand der Erfindung betrifft transgene Pflanzen enthaltend ein Kohlenhydrat-speicherndes Sink-Gewebe oder Mikroorganismen, die eine der erfindungsgemäßen Expressionskassetten oder erfindungsgemäßen Expressionsvektoren enthalten. Die Pflanze enthaltend ein Kohlenhydrat-speicherndes Sink-Gewebe oder der Mikroorganismus kann ausgewählt sein aus der Gruppe bestehend aus Bakterien, Hefen, Pilzen und pflanzlichen Organismen oder von diesen abgeleitete Zellen, Zellkulturen, Teile, Gewebe, Organe oder Vermehrungsgut, bevorzugt ist der pflanzliche Organismus ausgewählt aus der Gruppe der landwirtschaftlichen Nutzpflanzen. In besagten Pflanzen ist die Expression der transgen zu exprimierenden Nukleinsäuresequenz in mindesten einem Kohlenhydrat-speichernden (z.B. der Kartoffelknolle oder der Tomatenfrucht), -synthetisierenden oder -metabolisierenden Sink-Gewebe, unter ansonsten unveränderten Bedingungen höher als in einem anderen Gewebe.

Ein weiterer Gegenstand der Erfindung betrifft daher eine isolierte Nukleinsäuresequenz umfassend den Promotor der plastidären 1,4-α-D-Glukanphosphat-α-D-glucosyltransferase aus Vicia faba gemäß SEQ ID NO: 1, oder ein Fragment von desselben mit einer Länge von mindestens 500 Basenpaaren und mit im wesentlichen der gleichen Promotoraktivität wie die Promotorsequenz gemäß SEQ ID NO: 1 und die unter ansonsten unveränderten Bedingungen eine Transkription in mindestens einem Kohlenhydrat-speichernden, - synthetisierenden oder -metabolisierenden Sink-Gewebe vermittelt, die höher ist als in einem anderen Gewebe.

In einer bevorzugten Ausführungsform umfasst die erfindungsgemäße Nukleinsäuresequenz bzw. die erfindungsgemäße Expressionskassette eine Promotorsequenz
i) gemäß SEQ ID NO: 1 oder
ii) eine Promotorsequenz, die eine Homologie von mindestens 80% über einen Sequenzabschnitt von mindestens 500 Basenpaaren zu der Sequenz gemäß SEQ ID NO: 1 aufweist und die unter ansonsten unveränderten Bedingungen eine Transkription in mindestens einem Kohlenhydrat-speichernden, -synthetisierenden oder - metabolisierenden Sink-Gewebe vermittelt, die höher ist als in einem anderen Gewebe, oder
iii) Fragmente der Promotorsequenz gemäß i) mit einer Länge von mindestens 500 Basenpaaren und im wesentlichen der gleichen Promotoraktivität wie die Promotorsequenz gemäß SEQ ID NO: 1 und die die unter ansonsten unveränderten Bedingungen eine Transkription in mindestens einem Kohlenhydrat-speichernden, -synthetisierenden oder -metabolisierenden Sink-Gewebe vermittelt, die höher ist als in einem anderen Gewebe,
zudem noch die Sequenz kodierend für die 5'-untranslatierte Region des Phol-Gens aus Vicia faba. Besonders bevorzugt ist die durch SEQ ID NO: 2 beschriebene Sequenz.

In einer weiterhin bevorzugten Ausführungsform umfasst die erfindungsgemäße Nukleinsäuresequenz bzw. die erfindungsgemäße Expressionskassette eine Promotorsequenz
i) gemäß SEQ ID NO: 1 oder
ii) eine Promotorsequenz, die eine Homologie von mindestens 80% über einen Sequenzabschnitt von mindestens 500 Basenpaaren zu der Sequenz gemäß SEQ ID NO: 1 aufweist und die unter ansonsten unveränderten Bedingungen eine Transkription in mindestens einem Kohlenhydrat-speichernden, -synthetisierenden oder - metabolisierenden Sink-Gewebe vermittelt, die höher ist als in einem anderen Gewebe, oder
iii) Fragmente der Promotorsequenz gemäß i) mit einer Länge von mindestens 500 Basenpaaren und im wesentlichen der gleichen Promotoraktivität wie die Promotorsequenz gemäß SEQ ID NO: 1 und die die unter ansonsten unveränderten Bedingungen eine Transkription in mindestens einem Kohlenhydrat-speichernden, -synthetisierenden oder -metabolisierenden Sink-Gewebe vermittelt, die höher ist als in einem anderen Gewebe,
und zudem noch die Sequenz kodierend für die 5'-untranslatierte Region des Phol-Gens aus Vicia faba und eine Sequenz kodierend für ein Transitpeptid, bevorzugt für das Transitpeptid des Phol-Proteins aus Vicia faba gemäß SEQ ID NO: 8. Bevorzugt ist diese Sequenz in 3'-Richtung in Bezug auf einen der erfindungsgemäßen Promotoren orientiert. Als Promotorsequenz in diesem Zusammenhang besonders bevorzugt ist die durch SEQ ID NO: 3 beschriebene Sequenz.

Ein weiterer Gegenstand betrifft die Verwendung der erfindungsgemäßen isolierten Nukleinsäuresequenzen, Expressionsvektoren oder transgenen Pflanzen enthaltend ein Kohlenhydrat-speicherndes Sink-Gewebe zur transgenen Expression von Nukleinsäuren und/oder Proteinen in Pflanzen enthaltend ein Kohlenhydrat-speicherndes Sink-Gewebe. Insbesonders bevorzugt ist die Verwendung der besagten transgenen Pflanzen enthaltend ein Kohlenhydrat-speicherndes Sink-Gewebe oder von diesen abgeleitete Zellen, Zellkulturen, Teile, Gewebe, Organe oder Vermehrungsgut zur Herstellung von Nahrungs-, Futtermitteln, Saatgut, Pharmazeutika oder Feinchemikalien, wobei die Feinchemikalien bevorzugt Enzyme, Vitamine, Aminosäuren, Zucker, gesättigte oder ungesättigte Fettsäuren, natürliche oder synthetische Geschmacks-, Aroma- oder Farbstoffe sind. Erfindungsgemäß umfasst sind ferner Verfahren zur Herstellung besagter Nahrungs-, Futtermitteln, Saatgut, Pharmazeutika oder Feinchemikalien unter Einsatz der erfindungsgemäßen transgenen Pflanzen oder von diesen abgeleitete Zellen, Zellkulturen, Teile, Gewebe, Organe oder Vermehrungsgut.

Überraschenderweise zeigt der Promotor der plastidären 1,4-α-D-Glukanphosphat-α-D-glucosyltransferase (infolge "Phol-Promotor") aus der Ackerbohne *Vicia faba* eine starke und selektive Expression in Kohlenhydrat-speichernden Sink-Geweben wie den Kartoffelknollen, Rüben und in Früchten z. B. der Tomate. So kann beispielsweise eine starke Expression in Kartoffelknolle und Tomatenfrucht gefunden werden, während im Samen von Raps oder Arabidopsis keine erkennbare Expressionsaktivität detektiert werden konnte. Die Expressionsaktivität korreliert überraschend stark mit dem Ort/Ausmaß der Stärkebiosynthese bzw. dem Stärkegehalt.

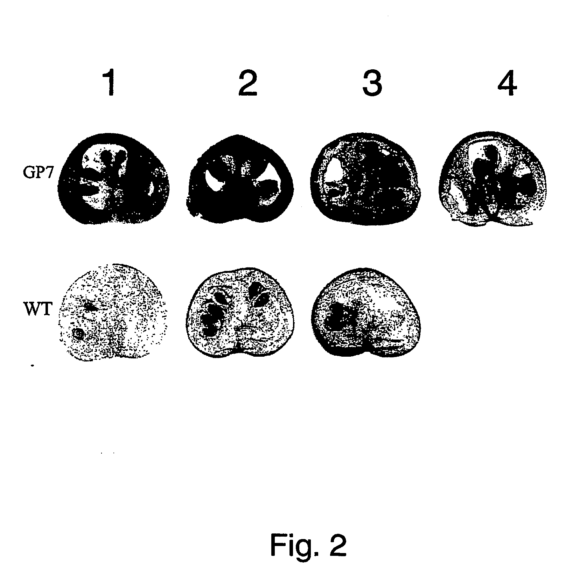

Der erfindungsgemäße Promotor weißt keinerlei Homologie zu dem bekannten Promotor einer putativen Stärkephosphorylase aus Kartoffel auf (St-Pierre B & Brisson N (1995) Plant Science 110:193-203; St-Pierre et al. (1996) Plant Mol Biol 30:1087-1098). Der Promotor der Stärkephosphorylase aus Kartoffel weißt neben einer Aktivität in den Knollen eine im Vergleich dazu bis zu 1,5 fach höhere Aktivität in den Wurzeln auf. Ebenfalls wurden hohe Aktivitäten in den Blattstielen und im Spross gefunden. Insgesamt war die Aktivität in den Knollen geringer als in Blattstielen, im Sproß, in den Stolonen und in den Wurzeln. Der erfindungsgemäße Phol-Promotor aus Vicia faba, hat hier gegenüber den Vorteil, dass neben der überraschend hohen Aktivität in den Kartoffelknollen und in den Tomatenfrüchten, nur geringe bis keine Aktivitäten in anderen Geweben gefunden wurden. Fig. 2 zeigt die gewebespezifische Expression des Phol-Promotors während der Fruchtentwicklung in Tomaten. In den Blättern und Wurzeln wurde nur eine geringe Aktivität detektiert. Der Promotor hat seine höchste Aktivität in jungen grünen Früchten, die mit der Fruchtreifung abnimmt.

Im Vergleich wurde der USP-Promotor (Bäumlein et al. (1991) Mol Gen Genet 225:459-467) untersucht. überraschenderweise zeigte sich in β-Glucuronidase (GUS) Expressionsexperimenten nur für den Phol-Promotor eine hohe Expression in den Kartoffelknollen und in den Tomatenfrüchten, während der USP-Promotor keine Expression aufwies. Eine inhärente Kopplung zwischen Expression in Samen und Kohlenhydrat-speichernden Sinkgewebe liegt demnach nicht vor.

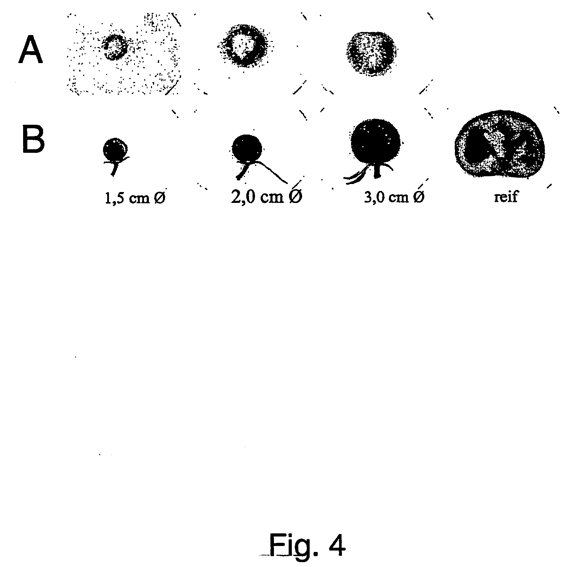

Die Expression durch den Phol-Promotor ist insbesondere in unreifen, grünen Früchten hoch und nimmt in den reifenden, roten Früchten ab. Dabei korreliert die Expressionshöhe mit dem Stärkegehalt (vgl. Fig.2 vs. Fig.4). Auch in Kartoffeln kann eine entsprechende Korrelation gesehen werden, wobei hier jedoch die Stärke erst in der adulten Knolle akkumuliert wird und der Promotor dort seine maximale Aktivität zeigt. In anderen Geweben konnte keine signifikante Expression nachgewiesen werden.

Aufgrund seiner hohen Expressionsaktivität und Spezifität ist der erfindungsgemäße Phol-Promotor von besonderem Wert für die Pflanzenbiotechnologie. Es kann erwartet werden, dass der Phol-Promotor in kohlenhydrat- und/oder speicherstarke-haltigen Geweben auch anderer Pflanzen aktiv ist. Insbesondere seine früh einsetzende Aktivität während der Entwicklung der Organe kann vorteilhaft zur Steigerung der Qualität der sich entwickelnden Frucht genutzt werden. Ganz besonders vorteilhaft ist hier die Verwendung in Ansätzen, die der Modifikation der Kohlenhydrat- und/oder Stärkebioaynthese oder des Kohlenhydrat- und/oder Stärkemetabolismus dienen. Beispiele für in diesem Rahmen bevorzugt zu exprimierenden Nukleinsäuresequenzen sind unten gegeben.

"Expression" meint die Transkription der transgen zu exprimierenden Nukleinsäuresequenz, kann aber - im Falle eines offenen Leserasters in "sense"-Orientierung - auch die Translation der transkribierten RNA der transgen zu exprimierenden Nukleinsäuresequenz in ein korrespondierendes Polypeptid mit einschließen.

"Transgen" meint - beispielsweise in Bezug auf einen transgenen Organismus oder Verfahren zur transgenen Expression von Nukleinsäuren - alle solche durch gentechnische Methoden zustande gekommene Konstruktionen oder Verfahren unter Verwendung derselben, in denen entweder
a) der Phol-Promotor gemäß SEQ ID NO: 1, 2 oder 3, ein Fragment des vorgenannten, mit einer Länge von mindestens 500 Basenpaaren und im wesentlichen der gleichen Promotoraktivität wie die Promotorsequenz gemäß SEQ ID NO: 1 und die die unter ansonsten unveränderten Bedingungen eine Transkription in mindestens einem Kohlenhydrat-speichernden, - synthetisierenden oder -metabolisierenden Sink-Gewebe vermittelt, die höher ist als in einem anderen Gewebe, oder
b) die transgen zu exprimierende Nukleinsäuresequenz in funktioneller Verknüpfung mit einem Promotor gemäß a), oder
c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Bevorzugt ist die in den Expressionskassetten enthaltene erfindungsgemäße Promotorsequenz (z.B. die Sequenz gemäß SEQ ID NO: 1, 2 oder 3) heterolog in Bezug auf die mit ihr funktionell verknüpfte, transgen zu exprimierende weitere Nukleinsäuresequenz. "Heterolog" meint in diesem Zusammenhang, dass die weitere Nukleinsäuresequenz nicht für das Gen kodiert, das natürlicherweise unter der Kontrolle des besagten Promotors steht.

"Natürliche genetische Umgebung" meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommendes Expressionskonstrukt - beispielsweise die natürlich vorkommende Kombination des Promotors gemäß SEQ ID NO: 1 und der kodierenden Sequenz des Gens der 1,4-α-D-Glukanphosphat-α-D-glucosyltransferase aus der Ackerbohne *Vicia faba* wird zu einem Expressionskonstrukt, wenn diese durch nicht-natürliche, synthetische ("künstliche") Verfahren wie beispielsweise einer in vitro Mutagenisierung geändert wird. Entsprechende Verfahren sind beschrieben (US 5,565,350; WO 00/15815; siehe auch oben).

"Transgen" meint in Bezug auf eine Expression ("transgene Expression") bevorzugt all solche unter Einsatz einer Expressionskassette, Expressionsvektor oder transgenen Pflanze enthaltend ein Kohlenhydrat-speicherndes Sink-Gewebe oder transgenen Mikroorganismus - entsprechend den oben gegebenen Definitionen - realisierten Expressionen.

"Gezielt" meint dabei in Bezug auf die Expression in kohlenhydrat-speichernden Sink-Geweben, dass die Expression unter Kontrolle eines der erfindungsgemäßen Promotoren in den kohlenhydrat-speichernden Sink-Geweben bevorzugt mindestens das zehnfache, ganz besonders bevorzugt mindestens das fünfzigfache, am meisten bevorzugt mindestens das hundertfache beträgt als in einem anderen Gewebe, bevorzugt in einem Source-Gewebe wie beispielsweise den Blättern.

Die erfindungsgemäßen Expressionskassetten, die von ihnen abgeleiteten Expressionsvektoren und transgenen Pflanzen, enthaltend ein Kohlenhydrat-speicherndes Sink-Gewebe, oder transgenen Mikroorganismen können funktionelle Äquivalente zu den unter SEQ ID NO: 1 beschriebenen Phol-Promotorsequenz umfassen. Unter funktionellen Äquivalenten wird hierin verstanden, dass sie eine Homologie von mindestens 80 %, ganz besonders bevorzugt mindestens 90 % über einen Sequenzabschnitt von mindestens 500 Basenpaaren, am meisten bevorzugt über die gesamte Sequenzlänge zu der Sequenz gemäß SEQ ID NO: 1 aufweisen, und die unter ansonsten unveränderten Bedingungen eine Transkription in mindestens einem Kohlenhydrat-speichernden, -synthetisierenden oder - metabolisierenden Sink-Gewebe vermitteln, die höher ist als in einem anderen Gewebe.

Fragmente von Promotoren - beispielsweise des Phol-Promotors beschrieben durch SEQ ID NO: 1 oder eines funktionellen Äquivalentes derselben - können eine Länge von mindestens 100 Basenpaare, oder mindestens 200 Basenpaare aufweisen.

Die erfindungsgemäßen Expressionskassetten, die von ihnen abgeleiteten Expressionsvektoren sowie transgenen Pflanzen, enthaltend ein Kohlenhydrat-speicherndes Sink-Gewebe, oder transgenen Mikroorganismen können erfindungsgemäße Fragmente des Promotors gemäß SEQ ID NO: 1 oder eines erfindungsgemäß funktionellen Äquivalentes desselben umfassen.

Zur Herstellung entsprechender Fragmente können beispielsweise nicht-essentielle Sequenzen eines erfindungsgemäßen Promotors deletiert werden, ohne die genannten wesentlichen Eigenschaften signifikant zu beeinträchtigen. Derartige Deletionsvarianten stellen erfindungsgemäße Fragmente des Phol-Promotors beschrieben durch SEQ ID NO: 1 oder eines erfindungsgemäß funktionellen Äquivalentes desselben dar.

Die Eingrenzung der Promotorsequenz auf bestimmte, essentielle regulatorische Regionen kann z.B. mit Hilfe von Suchroutinen zur Suche von Promotorelementen vorgenommen werden. Oft sind in den für die Promotoraktivität relevanten Regionen bestimmte Promotorelemente gehäuft vorhanden. Diese Analyse kann beispielsweise mit Computerprogrammen wie dem Programm PLACE ("Plant Cis-acting Regulatory DNA Elements") vorgenommen werden (Higo K et al. (1999) Nucl Acids Res 27(1): 297-300) oder der BIOBASE Datenbank "Transfac" (Biologische Datenbanken GmbH, Braunschweig).

Erfindungsgemäße Fragmente eines der erfindungsgemäßen Promotoren - beispielsweise des Phol-Promotors beschrieben-durch SEQ ID NO: 1 oder eines erfindungsgemäßen funktionellen Äquivalentes derselben - umfassen mindestens 500 Basenpaare der erfindungsgemäßen Promotoren und haben im wesentlichen der gleichen Promotoraktivität wie die Promotorsequenz gemäß SEQ ID NO: 1. und vermitteln unter ansonsten unveränderten Bedingungen eine Transkription in mindesten mindestens einem Kohlenhydrat-speichernden, -synthetisierenden oder -metabolisierenden Sink-Gewebe, die höher ist als in einem anderen Gewebe.
In einer bevorzugten Ausführungsform umfasst das erfindungsgemäße Fragment bevorzugt den 3'-Bereich des Phol-Promotors beschrieben durch SEQ ID NO: 1 oder eines erfdingungsgemäß funktionellen Äquivalentes derselben, wobei die jeweils bevorzugte Fragmentlänge vom Transkriptionsstart oder Translationsstart ("ATG"-Kodon) in 5'-Richtung stromaufwärts gerechnet wird.

Eine Promotoraktivität wird im wesentlichen als gleich bezeichnet, wenn die Transkription eines bestimmten zu exprimierenden Gens unter Kontrolle von z.B. eines erfindungsgemäßen Fragmentes der durch SEQ ID NO: 1 beschriebenen Phol-Promotorsequenz unter ansonsten unveränderten Bedingungen - in einem Kohlenhydrat-speichernden, -synthetisierenden oder -metabolisierenden Sink-Gewebe (wie z.B. der Kartoffelknolle, Rübe und Tomatenfrucht), ganz besonders bevorzugt in einem Stärke-speichernden, - synthetisierenden oder -metabolisierenden Sink-Gewebe (wie z.B. der Kartoffelknolle), höher ist als in einem anderen Gewebe, beispielsweise einem source-Gewebe.

"Kohlenhydrat" meint in diesem Zusammenhang bevorzugt Stärke oder Saccharose, besonders bevorzugt Stärke.

"Source-Gewebe" meint photosynthetisch aktive Gewebe. "Sink-Gewebe" meint Gewebe, die Nettoimporteure von photosynthetisch fixiertem Kohlendioxid sind und in oder Regel nicht photosynthetisch aktiv sind. Beispielhaft seien für Sink-Gewebe zu nennen: Wurzeln, Früchte, Knollen und Samenkörner.

Stärke-speichernde Sink-Gewebe (infolge "Stärke-Sink-Gewebe") meint bevorzugt solche Gewebe, die
a) photosynthetisch nicht selber aktiv sind und
b) zu zumindest einem Zeitpunkt ihrer Entwicklung einen Stärkegehalt aufweisen, der mittels eines Stärkenachweises nachweisbar ist. Bevorzugt ist als Stärkenachweis eine Färbung mit Lugol'scher Lösung (Lugol'sche Lsg.: z.B. 2 g KJ in 5 ml Wasser lösen, darin 1 g Jod lösen und 300 ml Wasser dazugegeben). Die Färbung erfolgt bis zu einem erkennbaren Blauton (ca. 15 min bei RT) und kann durch Waschen mit Wasser abgestoppt werden.

Dabei beträgt die Expression unter Kontrolle eines der erfindungsgemäßen Promotoren in einem Kohlenhydrat-speichernden, - synthetisierenden oder -metabolisierenden Sink-Gewebe oder einem Stärke-Sink-Gewebe, bevorzugt mindestens das doppelte, ganz besonders bevorzugt mindestens das fünffache, am meisten bevorzugt mindestens das zehnfache als in einem anderen Gewebe, beispielsweise einem Source-Gewebe.

Bevorzugt werden im Rahmen der Ermittlung der Expressionshöhe solche Sequenzen eingesetzt, die für leicht quantifizierbare Proteine kodieren. Ganz besonders bevorzugt sind dabei Reporter-proteine (Schenborn E, Groskreutz D. (1999) Mol Biotechnol 13(1): 29-44) wie "green fluorescence protein" (GFP) (Chui WL et al. (1996) Curr Biol 6:325-330; Leffel SM et al. (1997) Biotechniques 23(5):912-8), Chloramphenicoltransferase, Luziferase (Millar et al. (1992) Plant Mol Biol Rep 10:324-414), β-Glucuronidase oder β-Galactosidase. Ganz besonders bevorzugt ist die β-Glucuronidase (Jefferson et al. (1987) EMBO J 6:3901-3907).

"Ansonsten unveränderte Bedingungen" bedeutet, dass die Expression, die durch eine der zu vergleichenden Expressionskassetten initiiert wird, nicht durch Kombination mit zusätzlichen genetischen Kontrollsequenzen, zum Beispiel Enhancer-Sequenzen, modifiziert wird. Unveränderte Bedingungen bedeutet ferner, dass alle Rahmenbedingungen wie beispielsweise Pflanzenart, Entwicklungsstadium der Pflanzen, Zuchtbedingungen, Assaybedingungen (wie Puffer, Temperatur, Substrate etc.) zwischen den zu vergleichenden Expressionen identisch gehalten werden.

Die Expressionshöhe eines erfindungsgemäßen funktionell äquivalenten Promotors kann sowohl nach unten als auch nach oben im Vergleich zu dem Promotor gemäß SEQ ID NO: 1 abweichen. Bevorzugt sind dabei solche Sequenzen, deren Expressionshöhe, gemessen anhand der transkribierten mRNA oder dem infolge translatierten Protein, unter ansonsten unveränderten Bedingungen quantitativ um nicht mehr als 50 %, bevorzugt 25 %, besonders bevorzugt 10 % von einem Vergleichswert erhalten mit denen durch SEQ ID NO: 1 beschriebenen Promotoren unterscheidet. Besonders bevorzugt sind solche Sequenzen, deren Expressionshöhe, gemessen anhand der transkribierten mRNA oder dem infolge translatierten Protein, unter ansonsten unveränderten Bedingungen quantitativ um mehr als 50 %, bevorzugt 100 %, besonders bevorzugt 500 %, ganz besonders bevorzugt 1000 % einen Vergleichswert erhalten mit dem durch SEQ ID NO: 1 beschriebenen Promotor übersteigt. Bevorzugt ist als Vergleichswert die Expressionshöhe der natürlicherweise von dem Promotor exprimierten mRNAs einer 1,4-α-D-Glukanphosphat-α-D-glucosyltransferase oder des daraus resultierenden Proteins. Bevorzugt ist ferner als Vergleichswert die Expressionshöhe erhalten mit einer beliebigen, aber bestimmten Nukleinsäuresequenz, bevorzugt solchen Nukleinsäuresequenzen, die für leicht quantifizierbare Proteine kodieren. Ganz besonders bevorzugt sind dabei Reporter-Proteine (Schenborn E & Groskreutz D (1999) Mol Biotechnol 13(1):29-44) wie das "green fluorescence protein" (GFP) (Chui WL et al. (1996) Gurr Biol 6:325-330; Leffel SM et al. (1997) Biotechniques. 23(5);912-8), die Chloramphenicoltransferase, eine Luziferase (Millar et al. (1992) Plant Mol Biol Rep 10:324-414) oder die β-Glucuronidase, ganz besonders bevorzugt ist die β-Glucuronidase (Jefferson et al. (1987) EMBO J 6:3901-3907).

Erfindungsgemäß funktionelle Äquivalente umfassen auch natürliche oder künstliche Mutationen der unter SEQ ID NO: 1 beschriebenen Promotorsequenz.

Mutationen umfassen Substitutionen, Additionen, Deletionen, Inversionen oder Insertionen eines oder mehrerer Nukleotidreste. Somit werden beispielsweise auch solche Nukleotidsequenzen durch die vorliegende Erfindung mit umfasst, welche man durch Modifikation des Phol-Promotors gemäß SEQ ID NO: 1 erhält. Ziel einer solchen Modifikation kann die weitere Eingrenzung der darin enthaltenen Sequenz oder z.B. auch das Einfügen oder Entfernen von Restriktionsendonukleaseschnittstellen, die Entfernung überflüssiger DNA oder das Hinzufügen weiterer Sequenzen, zum Beispiel weiterer regulatorischer Sequenzen, sein.

Wo Insertionen, Deletionen oder Substitutionen, wie z.B. Transitionen und Transversionen, in Frage kommen, können an sich bekannte Techniken, wie in vitro-Mutagenese, "primer repair", Restriktion oder Ligation verwendet werden. Transition meint einen Basenpaaraustausch eines Purin/Pyrimidin-Paares in ein anderes Purin/Pyrimidin-Paar (z.B. A-T gegen G-C). Transversion meint einen Basenpaaraustausch eines Purin/Pyrimidin-Paares gegen ein Pyrimidin/Purin-Paar (z.B. A-T gegen T-A). Deletion meint die Entfernung eines oder mehrerer Basenpaare. Insertion meint die Einführung eines oder mehrerer Basenpaare.

Durch Manipulationen, wie z.B. Restriktion, "chewing-back" oder Auffüllen von Überhängen für "blunt ends" können komplementäre Enden der Fragmente für die Ligation zur Verfügung gestellt werden. Zu analogen Ergebnissen kann man auch unter Verwendung der Polymerasekettenreaktion (PCR) unter Verwendung spezifischer Oligonukleotid-Primer kommen.

Unter Homologie zwischen zwei Nukleinsäuren wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2,912 | Average Mismatch:-2,003 |

Beispielhaft wird unter einer Sequenz, die eine Homologie von mindestens 50 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 50 % aufweist.

Funktionelle Äquivalente meint ferner DNA Sequenzen, die unter Standardbedingungen mit der Nukleinsäuresequenz kodierend für den Pho1 Promotor gemäß SEQ ID NO: 1 oder der zu ihr komplementären Nukleinsäuresequenzen hybridisieren und die im wesentlichen gleichen Promotoreigenschaften haben. Der Begriff der Standardhybridisierungsbedingungen ist breit zu verstehen und meint sowohl stringente als auch weniger stringente Hybridisierungsbedingungen. Solche Hybridisierungsbedingungen sind unter anderem bei Sambrook J, Fritsch EF, Maniatis T et al., in Molecular Cloning - A Laboratory Manual, 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6. beschrieben.

Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0,2X SSC bei 50°C bevorzugt bei 65°C) (20X SSC: 0,3 M Natriumcitrat, 3 M NaCl, pH 7.0). Darüber hinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridsierung bevorzugt bei 42°C ausgeführt. Einige beispielhafte Bedingungen für Hybridisierung und Waschschritte sind infolge gegeben:

### (1) Hybridisierungsbedingungen mit zum Beispiel

a) 4X SSC bei 65°C, oder
b) 6X SSC, 0,5% SDS, 100 µg/ml denaturierte, fragmentierte Lachssperma-DNA bei 65°C, oder
c) 4X SSC, 50% Formamid, bei 42°C, oder
d) 2X oder 4X SSC bei 50°C (schwach stringente Bedingung), oder
e) 2X oder 4X SSC, 30 bis 40 % Formamid bei 42°C (schwach stringente Bedingung), oder
f) 6x SSC bei 45°C, oder,
g) 0,05 M Natriumphosphatpuffer pH 7,0, 2 mM EDTA, 1% BSA und 7% SDS.

### (2) Waschschritte mit zum Beispiel

a) 0,1X SSC bei 65°C, oder
b) 0,1X SSC, 0,5 % SDS bei 68°C, oder
c) 0,1X SSC, 0,5 % SDS, 50% Formamid bei 42°C, oder
d) 0,2X SSC, 0,1 % SDS bei 42°C, oder
e) 2X SSC bei 65°C (schwach stringente Bedingung), oder
f) 40 mM Natriumphosphatpuffer pH 7,0, 1% SDS, 2 mM EDTA.

Verfahren zur Herstellung erfindungsgemäßer funktioneller Äquivalente umfassen bevorzugt die Einführung von Mutationen in den Pho1 Promotor gemäß SEQ ID NO: 1. Eine Mutagenese kann ungerichtet ("random") erfolgen, wobei die mutagenisierten Sequenzen anschließend bezüglich ihrer Eigenschaften nach einer "trial-anderror" Prozedur durchmustert werden. Besonders vorteilhafte Selektionskriterien umfassen beispielsweise die Höhe der resultierenden Expression der eingeführten Nukleinsäuresequenz in einem Stärke-Sink-Gewebe.

Verfahren zur Mutagenisierung von Nukleinsäuresequenzen sind dem Fachmann bekannt und schließen beispielhaft die Verwendung von Oligonukleotiden mit einer oder mehr Mutationen im Vergleich zu der zu mutierenden Region ein (z.B. im Rahmen einer "Sitespecific mutagenesis"). Typischerweise kommen Primer mit ungefähr 15 bis ungefähr 75 Nukleotiden oder mehr zum Einsatz, wobei bevorzugt ca. 10 bis ca. 25 oder mehr Nukleotidreste an beiden Seiten der zu verändernden Sequenz lokalisiert sind. Details und Durchführung besagter Mutageneseverfahren sind dem Fachmann geläufig (Kunkel et al. (1987) Methods Enzymol 154:367-382; Tomic et al. (1990) Nucl Acids Res 12:1656; Üpender et al. (1995) Biotechniques 18(1):29-30; US 4,237,224). Eine Mutagenese kann auch durch Behandlung von beispielsweise Expressionsvektoren, die eine der erfindungsgemäßen Nukleinsäuresequenzen enthalten, mit mutagenisierenden Agentien wie Hydroxylamin realisiert werden.

Unter einer funktionellen Verknüpfung versteht man zum Beispiel die sequentielle Anordnung eines der erfindungsgemäßen Promotoren, der transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsburesequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierenden Nukleinaauresequenz hinter der als Promotor fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer Expressionskassette bzw. einer funktionellen Verknüpfung kann mittels gängiger Rekombinations- und Klonierungstechniken realisiert werden, wie sie beispielsweise in Maniatis T et al. (1989) Molecular Cloning: A Laboratory Manual, Cold Spring Harbor Laboratory, Cold Spring Harbor, NY sowie in Silhavy TJ et al.(1984) Experiments with Gene Fusions, Cold Spring Harbor Laboratory, Cold Spring Harbor, NY und in Ausubel FM et al. (1987) Current Protocols in Molecular Biology, Greene Publishing Assoc. and Wiley Interscience beschrieben sind. Ein in diesem Rahmen geeignetes Verfahren ist beispielsweise die auf Rekombination basierende GATEWAY^{™}-Cloning Technologie (Invitrogen Inc.).

Die Herstellung einer erfindungsgemäßen Expressionskassette erfolgt beispielsweise durch Fusion eines erfindungsgemäßen Promotors gemäß SEQ ID NO: 1 (oder eines erfindungsgemäßen funktionellen Äquivalentes oder eines erfindungsgemäßen Fragments) mit einer transgen zu exprimierenden Nukleotidsequenz, gegebenenfalls einer für ein Transitpeptid kodierenden Sequenz, vorzugsweise ein chloroplastenspezifisches Transitpeptid, welches vorzugsweise zwischen dem Promotor und der jeweiligen Nukleotidsequenz angeordnet ist, sowie optional einem Terminator- oder Polyadenylierungssignal. Die Kombination des Pho1 Promotors mit der Sequenz kodierend für sein natürliches Transitpeptid (entsprechend SEQ ID NO: 3) ist dabei besonders bevorzugt.

Unter einer Expressionskassette sind aber auch solche Konstruktionen zu verstehen, bei denen einer der erfindungsgemäßen Promotoren, ohne dass er zuvor mit einer transgen zu exprimierenden Nukleinsäuresequenz funktionell verknüpft wurde, zum Beispiel über eine gezielte homologe Rekombination oder eine zufällige Insertion in ein Wirtsgenom eingeführt wird, dort regulatorische Kontrolle über mit ihm dann funktionell verknüpfte Nukleinsäuresequenzen übernimmt und die transgene Expression derselben steuert. Durch Insertion des Promotors - zum Beispiel durch eine homologe Rekombination - vor eine für ein bestimmtes Polypeptid kodierende Nukleinsäure erhält man eine erfindungsgemäße Expressionskassette, die die Expression des bestimmten Polypeptides in der Pflanze steuert. Ferner kann die Insertion des Promotors auch derart erfolgen, dass antisense-RNA zu der für ein bestimmtes Polypeptid kodierenden Nukleinsäure exprimiert wird. Damit wird die Expression des bestimmten Polypeptides in Pflanzen herunterreguliert oder ausgeschaltet.

Die in den erfindungsgemäßen Expressionskassetten enthaltenen transgen zu exprimierenden Nukleinsäuresequenzen können mit weiteren genetischen Kontrollsequenzen neben einem der erfindungsgemäßen Promotoren funktionell verknüpft sein.

Der Begriff der genetischen Kontrollsequenzen ist breit zu verstehen und meint all solche Sequenzen, die einen Einfluss auf das Zustandekommen oder die Funktion der erfindungsgemäßen Expressionskassette haben. Genetische Kontrollsequenzen modifizieren zum Beispiel die Transkription und/oder Translation in prokaryotischen oder eukaryotischen Organismen. Vorzugsweise umfassen die erfindungsgemäßen Expressionskassetten 5'-stromaufwärts von der jeweiligen transgen zu exprimierenden Nukleinsäuresequenz einen der erfindungsgemäßen Promotoren und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH (1991) J Biol Chem 266(26):17131-17135) und Hitzestress (Schöffl F et al. (1989) Mol Gen Genet 217(2-3):246-53) beschrieben.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel *E.coli* Bakterien ermöglichen. Als Pflanzenpromotoren kommen im Prinzip alle oben beschriebenen Promotoren in Frage. Vorstellbar ist zum Beispiel, dass eine bestimmte Nukleinsäuresequenz durch einen Promotor (zum Beispiel einen der erfindungsgemäßen Promotoren) in einem Pflanzengewebe als sense-RNA transkribiert und in das entsprechende Protein translatiert wird, während die gleiche Nukleinsäuresequenz durch einen anderen Promotor mit einer anderen Spezifität in einem anderen Gewebe zu anti-sense-RNA transkribiert und das entsprechende Protein herunterreguliert wird. Dieses kann durch eine erfindungsgemäße Expressionskassette realisiert werden, indem der eine Promotor vor die transgen zu exprimierende Nukleinsäuresequenz positioniert wird und der andere Promotor dahinter.

Genetische Kontrollsequenzen umfassen ferner auch die 5'-untranslatierte Region, Introns, die nichtkodierende 3'-Region oder auch für Signal- oder Transitpeptide kodierende Sequenzen von Genen, bevorzugt des Phol-Gens. Es ist gezeigt worden, dass diese signifikante Funktionen bei der Regulation der Genexpression spielen können. So wurde gezeigt, dass 5'-untranslatierte Sequenzen die transiente Expression heterologer Gene verstärken können. Sie können ferner die Gewebespezifität fördern (Rouster J et al. (1998) Plant J 15:435-440). Umgekehrt unterdrückt die 5'-untranslatierte Region des opaque-2 Gens die Expression. Eine Deletion der entsprechenden Region führt zu einer Erhöhung der Genaktivität (Lohmer S et al. (1993) Plant Cell 5:65-73).

In transgenen Reiszellen führte die Verwendung des Actl-Introns in Kombination mit dem 35S-Promotor zu einer gegenüber dem isolierten 35S-Promotor um das Zehnfache gesteigerten Expressionsrate (McElroy et al. (1991) Mol Gen Genet 231(1):150-160). Eine Optimierung der Sequenzumgebung der Translations-Initiationsstelle des GUS-Reportergens resultierte in einer vierfachen Steigerung der GUS-Expression in transformierten Reiszellen. Eine Kombination der optimierten Translations-Initiationsstelle und des Act1-Introns resultierte in einer 40-fachen Steigerung der GUS-Expression durch den CaMV35S-Promotor in transformierten Reiszellen; ähnliche Ergebnisse wurden anhand von transformierten Maiszellen erzielt. Insgesamt wurde aus den zuvor beschriebenen Untersuchungen geschlussfolgert, dass die auf dem Actl-Promotor basierenden Expressionsvektoren dazu geeignet sind, eine hinreichend starke und konstitutive Expression von Fremd-DNA in transformierten Zellen monokotyler Pflanzen zu steuern.

Die unter SEQ ID NO: 2 oder 3 angegebenen Promotorsequenzen enthält den Abschnitt des Phol-Gens, der den Promotor und die 5'-untranslatierte Region bis vor das ATG-Startcodon des Phol-Proteins repräsentiert.

Die Expressionskassette kann vorteilhafterweise eine oder mehrere sogenannte "enhancer Sequenzen" funktionell verknüpft mit dem Promotor enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien in einer der erfindungsgemäßen Expressionskassetten enthalten sein.

Als Kontrollsequenzen sind weiterhin solche zu verstehen, die eine homologe Rekombination bzw. Insertion in das Genom eines wirtsorganismus ermöglichen oder die Entfernung aus dem Genom erlauben. Bei der homologen Rekombination kann zum Beispiel der natürliche Promotor eines bestimmten Gens gegen einen der erfindungsgemäßen Promotoren ausgetauscht werden. Einer der erfindungsgemäßen Promotoren kann - wie oben beschrieben - mittels homologer Rekombination vor ein transgen zu exprimierendes endogenes Zielgen platziert werden, indem der Promotor mit DNA-Sequenzen verknüpft wird, die zum Beispiel zu endogenen Sequenzen homolog sind, die dem Leseraster des Zielgens vorgelagert sind. Derartige Sequenzen sind als genetische Kontrollsequenzen zu verstehen. Methoden wie die cre/lox-Technologie erlauben eine gewebespezifische, unter Umständen induzierbare Entfernung der Expressionskassette aus dem Genom des Wirtsorganismus (Sauer B (1998) Methods (Duluth) 14(4):381-92). Hier werden bestimmte flankierende Sequenzen dem Zielgen angefügt (lox-Sequenzen), die später eine Entfernung mittels der cre-Rekombinase ermöglichen.

Zur Selektion erfolgreich homolog rekombinierte oder auch transformierter Zellen ist es in der Regel erforderlich, einen selektionierbaren Marker (s.u.) zusätzlich einzuführen. Homologe Rekombination ist ein relativ seltenes Ereignis in höheren Eukaryoten, vor allem in Pflanzen. Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben.

Als Kontrollsequenzen geeignete Polyadenylierungssignale sind pflanzliche Polyadenylierungssignale sowie - vorzugsweise - solche, die im wesentlichen T-DNA Polyadenylierungssignalen aus Agrobakterium tumefaciens entsprechen. In einer besonders bevorzugten Ausführungsform enthält die Expressionskassette eine in Pflanzen funktionelle Terminatorsequenz. In Pflanzen funktionelle Terminatorsequenzen meint allgemein solche Sequenzen, die in der Lage sind, in Pflanzen den Abbruch der Transkription einer DNA-Sequenz zu bewirken. Beispiele für geeignete Terminatorsequenzen sind der OCS (Octopin-Synthase)-Terminator und der NOS (Nopalin-Synthase)-Terminator. Besonders bevorzugt sind jedoch pflanzliche Terminatorsequenzen. Pflanzliche Terminatorsequenzen meint allgemein solche Sequenzen, die Bestandteil eines natürlichen pflanzlichen Gens sind. Besonders bevorzugt sind dabei der Terminator des Cathepsin D Inhibitor Gens aus Kartoffel (GenBank Acc. No.: X74985) oder der Terminator des Speicherproteingens VfLE1B3 (GenBank Acc. No.: Z26489) aus der Ackerbohne. Diese Terminatoren sind den im Stand der Technik beschriebenen viralen oder T-DNA Terminatoren mindestens gleichwertig.

Die transgene Expression der durch die Nukleinsäuresequenzen kodierten Proteine unter Kontrolle des Phol-Promotor ist in jedem gewünschten Zellkompartiment, wie z.B. dem Endomembransystem, der Vakuole und den Chloroplasten möglich. Durch Nutzung des sekretorischen Weges sind gewünschte Glykosylierungsreaktionen, besondere Faltungen u.ä. möglich. Die dafür als genetische Kontrollsequenzen notwendigen Signalpeptidsequenzen können sowohl bereits in einzelnen Expressionskassetten bereitgestellt werden, als auch durch Verwendung einer geeigneten Klonierungsstrategie gemeinsam mit der transgen zu exprimierenden Nukleinsäuresequenz in die Expressionskassette eingebracht werden.

Als Signal- oder Transitpeptidsequenzen können sowohl geneigene oder heterologe Sequenzen genutzt werden. Zusätzliche, heterologe zur funktionellen Verknüpfung bevorzugte aber nicht darauf beschränkte Sequenzen sind weitere Targeting-Sequenzen zur Gewährleistung der subzellulären Lokalisation im Apoplasten, in der Vakuole, in Plastiden, in Mitochondrien, im Endoplasmatischen Retikulum (ER), im Zellkern, in Ölkörperchen oder anderen Kompartimenten; sowie Translationsverstärker wie die 5'-Leadersequenz aus dem Tabak-Mosaik-Virus (Gallie et al. (1987) Nucl Acids Res 15:8693-8711) und dergleichen. Das Verfahren, an sich nicht in den Plastiden lokalisierte Proteine, gezielt in die Plastiden zu transportieren, ist beschrieben (Klosgen RB und Weil JH (1991) Mol Gen Genet 225(2):297-304; Van Breusegem F et al. (1998) Plant Mol Biol 38(3):491-496). Bevorzugte Sequenzen sind:
a) das Transitpeptid des Phol-Proteins,
b) das Transitpeptid der kleinen Untereinheit (SSU) der Ribulosebisphosphatcarboxylase (Rubisco ssu) aus beispielsweise Erbse, Mais oder Sonnenblume,
c) Transitpeptide abgeleitet von Genen der pflanzlichen Fettsaurebiosynthese wie das Transitpeptid des plastidären "Acyl Carrier Protein" (ACP), die Stearyl-ACP-Desaturase, β-Ketoacyl-ACP Synthase oder die Acyl-ACP-Thioesterase,
d) das Transitpeptid der GBSSI ("granula Bound Starch Synthase I")
e) das Transitpeptid der LHCP II Gene
f) das Transitpeptide der Transketolase (EP-A1 0 723 017).

Die Zielsequenzen können mit anderen, von dem Transitpeptid kodierenden Sequenzen verschiedenen, Targeting-Sequenzen verknüpft sein, um eine subzellulären Lokalisation im Apoplasten, in der Vakuole, in Plastiden, in Mitochondrien, im Endoplasmatischen Retikulum (ER), im Zellkern, in Ölkörperchen oder anderen Kompartimenten zu gewährleisten. Ferner können Translationsverstärker wie die 5'-Leadersequenz aus dem Tabak-Mosaik-Virus (Gallie et al. (1987) Nucl Acids Res 15:8693-8711) und dergleichen zum Einsatz kommen.

Wie bereits erwähnt ist - in einer besonders bevorzugten Ausführungsform - die Sequenz kodierend für ein Transitpeptid bereits in die Kassette oder den Vektor zur Herstellung einer Expressionskassette oder eines Expressionsvektors insertiert. Ganz besonders bevorzugt verwendet man dazu Konstrukte, die den Phol-Promotor in Verknüpfung mit einer Sequenz kodierend für das putative Transitpeptid des Phol-Proteins (SEQ ID NO: 8) umfassen. Eine in diesem Rahmen bevorzugt verwendetes Promotorkonstrukt ist durch SEQ ID NO: 3 beschrieben. Um eine ausreichende Prozessierung zu gewährleisten, umfaßt dieses Konstrukt Sequenzen kodierend für ungefähr weitere 10 Aminosäuren hinter dem Transitpeptid und gewährleistet so eine effiziente Prozessierung desselben. Ein in den Plastiden transgen zu exprimierende Protein würde dann in der dem Fachmann geläufigen Weise in das Leseraster hinter die Sequenz kodierend für das Transitpeptid kloniert werden, wodurch es zur transgenen Expression eines chimären Proteins mit dem Phol-Transitpeptid kommen würde.

Dem Fachmann ist eine Vielzahl von Nukleinsäuren bzw. Proteinen bekannt, deren Expression gesteuert durch die erfindungsgemäßen Expressionskassetten vorteilhaft ist. Ferner sind dem Fachmann eine Vielzahl von Genen bekannt, durch deren Reprimierung oder Ausschaltung mittels transgener Expression beispielsweise einer entsprechenden doppelsträngigen RNA oder einer antisense-RNA ebenfalls vorteilhafte Effekte erreicht werden können. Im Rahmen der vorliegenden Erfindung sind insbesondere solche Zielgene geeignet, die eine Rolle spielen im Zucker- oder Stärkemetabolismus, bei sink-source Relationen, bei der Balance von organischen Säuren, als Geschmackskomponenten, bei der Resistenz gegen biotische Stressfaktoren (Pathogene, Viren, Insekten und Krankheiten), bei der Resistenz gegen abiotische Stressfaktoren (Hitze, Kälte, Trockenheit, erhöhte Feuchtigkeit, Umweltgifte, UV-Strahlung), bei der Konsistenz der Gewebe oder bei Wasser/pH Verhältnissen, bei der Verbesserung von Nahrungs- oder Futtereigenschaften, die Verbesserung der Keimungs- und/oder Lagereigenschaften sowie bei der Verbesserung der Wachstumsrate oder des Ertrages.

Die Steigerung des Stärkegehaltes ist insbesondere für Tomate und Kartoffel von besonderem Interesse. Eine normale Tomate besteht zu etwa 80 bis 95% aus Wasser, während Stärke - als der eigentlich relevante Bestandteil für die Herstellung von beispielsweise Tomatenmark, Ketchup - nur einen geringen Anteil hat. Selbst eine geringfügige Erhöhung des Stärkeanteils wäre von erheblicher wirtschaftlicher Bedeutung. In frühen Reifestadien liegt der Stärkegehalt der Tomate mit 20% deutlich höher, sinkt dann jedoch während der Entwicklung durch Mobilisierung der Stärke und Umsetzung in Zucker ab. Bei Kartoffel wirkt sich ein erhöhter Stärkeanteil insbesondere vorteilhaft auf die Frittiereigenschaften aus.

Nachfolgend seien beispielhaft jedoch nicht einschränkend Nukleinsäurensequenzen genannt, deren Expression unter Kontrolle eines der erfindungsgemäßen Promotoren vorteilhafte Effekte bietet:
1. Verbesserter Schutz der Pflanze gegen abiotische Stressfaktoren wie Trockenheit, Hitze, oder Kälte zum Beispiel durch Überexpression von "antifreeze"-Polypeptiden aus Myoxocephalus Scorpius (WO 00/00512), Myoxocephalus octodecemspinosus, dem Arabidopsis thaliana Transkriptionsaktivator CBF1, Glutamatdehydzogenasen (WO 97/12983, WO 98/11240), Calciumabhängigen Proteinkinasegenen (WO 98/26045), Calcineurinen (WO 99/05902), Caseinkinase aus Hefe (WO 02/052012), Farnesyltransferasen (WO 99/06580; Pei ZM et al. (1998) Science 282: 287-290), Ferritin (Deak M et al. (1999) Nature Biotechnology 17:192-196), Oxalatoxidase (WO 99/04013; Dunwell JM (1998) Biotechn Genet Eng Rev 15:1-32), DREB1A-Faktor ("dehydration response element B 1A"; Kasuga M et al. (1999) Nature Biotech 17:276-286), Genen der Mannitol- oder Trehalosesynthese wie der Trehalosephosphatsynthase oder der Trehalosephosphatphosphatase (WO 97/42326) oder durch Inhibition von Genen wie der Trehalase (WO 97/50561).
2. Expression von Stoffwechselenzymen zur Verwendung im Futter- und Nahrungsmittelbereich z.B. von Phytasen und Cellulasen. Besonders bevorzugt sind Nukleinsäuren wie die künstliche für eine mikrobielle Phytase kodierende cDNA (GenBank Acc.-No.: A19451) oder funktionelle Äquivalente derselben.
3. Erreichen einer Resistenz zum Beispiel gegen Pilze, Insekten, Nematoden und Krankheiten durch gezielte Absonderung oder Anreicherung bestimmter Metaboliten oder Proteine. Beispielhaft seien genannt Glukosinolate (Abwehr von Herbivoren), Chitinasen oder Glukanasen und andere Enzyme, die die Zellwand von Parasiten zerstören, Ribosom-inaktivierende Proteine (RIPs) und andere Proteine der pflanzlichen Resistenz- und Stressreaktion, wie sie bei Verletzung oder mikrobiellem Befall von Pflanzen oder chemisch durch zum Beispiel Salicylsäure, Jasmonsäure oder Ethylen induziert werden, Lysozyme aus nicht-pflanzlichen Quellen wie zum Beispiel T4 Lysozym oder Lysozym aus verschiedenen Säugern, insektizide Proteine wie *Bacillus thuringiensis* Endotoxin, α-Amylaseinhibitor oder Proteaseinhibitoren (cowpea Trypsininhibitor), Lectine wie Weizenkeimagglutinin, RNAsen oder Ribozyme. Weitere Beispiele sind Nukleinsäuren, die für die chit42 Endochitinase aus Trichoderma harzianum (GenBank Acc.-No.: S78423) oder für das N-hydroxylierende, multifunktionelle Cytochrom P-450 (CYP79) Protein aus Sorghum bicolor (GenBank Acc.-No.: U32624) oder funktionelle Äquivalente derselben kodieren.
   Vorteilhaft ist die Akkumulation von Glukosinolaten zum Schutz vor Schädlingen (Rask L et al.(2000) Plant Mol Biol 42:93-113; Menard R et al. (1999). Phytochemistry 52:29-35), die Expression von *Bacillus thuringiensis* Endotoxinen (Vaeck et al. (1987) Nature 328:33-37) oder der Schutz gegen Pilzbefall durch Expression von Chitinasen z.B. aus der Bohne (Broglie et al. (1991) Science 254:1194-1197). Durch Expression des Lectins Agglutinin aus Schneeglöckchen (*Galanthus nivalis*) kann eine Resistenz gegen Schädlinge wie z.B. den Reisschädling *Nilaparvata lugens* in Reispflanzen erreicht werden (Rao et al. (1998) Plant J 15(4):469-77).
   Die Expression synthetischer cryIA(b) and cryIA(c) Gene, die für Lepidopteren-spezifische Δ-Endotoxine aus *Bacillus thuringiensis* kodieren, kann in verschiedenen Pflanzen eine Resistenz gegen Schadinsekten bewirken (Goyal RK et al. (2000) Crop Protection 19(5):307-312).
   Weiterhin zur Pathogen Abwehr geeignete Zielgene umfassen "Polygalacturonase inhibiting protein" (PGIP), Thaumatin, Invertase sowie antimikrobielle Peptide wie Lactoferrin (Lee TJ et al. (2002) J Amer Soc Horticult Sci 127(2): 158-164).
4. Expression von Genen, die eine Akkumulation von Feinchemikalien, wie von Tocopherolen, Tocotrienolen oder Carotinoiden bewirken. Beispielhaft sei die Phytoendesaturase genannt. Bevorzugt sind Nukleinsäuren, die für die Phytoendesaturase aus Narcissus pseudonarcissus (GenBank ACC.-NO.: X78815) oder funktionelle Äquivalente derselben kodieren.
5. Produktion von Nutraceuticals wie zum Beispiel polyungesättigten Fettsäuren (z.B. Arachidonsäure, Eicosapentaensäure oder Docosahexaensäure) durch Expression von Fettsäureelongasen und/oder -desaturasen oder Produktion von Proteinen mit verbessertem Nährwert wie zum Beispiel mit einem hohen Anteil an essentiellen Aminosäuren (z.B. das methioninreiche 2S Albumingen der Brasilnuss). Bevorzugt sind Nukleinsäuren, die für das methioninreiche 2S Albumin aus Bertholletia excelsa (GenBank Acc.-No.:AB044391), die Δ6-Acyllipiddesaturase aus Physcomitrella patens (GenBank Acc.-No.: AJ222980; Girke et al. (1998) Plant J 15:39-48), die Δ6-Desaturase aus Mortierella alpina (Sakuradani et al 1999 Gene 238:445- 453), die Δ5-Desaturase aus Caenorhabditis elegans (Michaelson et al. 1998, FEBS Letters 439:215-218), die Δ5-Fettsäuredesaturase (des-5) aus Caenorhabditis elegans (GenBank Acc.-No.: AF078796), die Δ5-Desaturase aus Mortierella alpina (Michaelson et al. JBC 273:19055 - 19059), die Δ6-Elongase aus Caenorhabditis elegans (Beaudoin et al. 2000, PNAS 97:6421-6426), die Δ6-Elongase aus Physcomitrella patens (Zank et al. 2000, Biochemical Society Transactions 28:654-657) oder funktionelle Äquivalente derselben kodierten.
6. Produktion von hochwertigen Proteinen und Enzymen zu industriellen Zwecken (wie beispielsweise Enzymen wie Lipasen) oder als Pharmazeutika (wie zum Beispiel Antikörpern, Blutgerinnungsfaktoren, Interferonen, Lymphokinen, "colony stimulation factor", Plasminogenaktivatoren, Hormonen oder Vakzinen wie beschrieben bei Hood EE, Jilka JM (1999) Curr Opin Biotechnol 10(4):382-6; Ma JK, Vine ND (1999) Curr Top Microbiol Immunol 236:275-92). Beispielsweise konnte rekombinantes Avidin aus Hühnereiweiß und bakterieller β-Glucuronidase (GUS) in großen Maßstab in transgenen Maispflanzen produziert werden (Hood et al. (1999) Adv Exp Med Biol 464:127-47. Review.).
8. Erreichen einer erhöhten Speicherfähigkeit in Zellen, die normalerweise weniger Speicherproteine oder -lipide enthalten mit dem Ziel, den Ertrag an diesen Substanzen zu erhöhen, zum Beispiel durch Expression eine Acetyl-CoA Carboxylase. Bevorzugt sind Nukleinsäuren, die für die Acetyl-CoA Carboxylase (Accase) aus Medicago sativa (GenBank Acc.-No.: L25042) oder funktionelle Äquivalente derselben kodieren.

Weitere Beispiele für vorteilhafte Gene sind zum Beispiel genannt bei Dunwell JM, Transgenic approaches to crop improvement, J Exp Bot. 2000;51 Spec No; Seite 487-96.

Ferner können funktionelle Analoga der genannten Nukleinsäuren bzw. Proteine exprimiert werden. Funktionelle Analoga meint hier all die Sequenzen, die im wesentlichen die gleiche Funktion haben d.h. zu der Funktion (zum Beispiel einer Substratumsetzung oder einer Signaltransduktion) befähigt sind wie auch das beispielhaft genannte Protein. Dabei kann das funktionelle Analogon sich in anderen Merkmalen durchaus unterscheiden. Es kann zum Beispiel eine höhere oder niedrigere Aktivität haben oder auch über weitere Funktionalitäten verfügen. Funktionelle Analoga meint ferner Sequenzen, die für Fusionsproteine bestehend aus einem der bevorzugten Proteine und anderen Proteinen zum Beispiel einem weiteren bevorzugten Protein oder aber auch einer Signalpeptidsequenz kodieren.

Dem Fachmann ist ferner bekannt, dass er die oben beschriebenen Gene nicht direkt unter Verwendung der für diese Gene kodierenden Nukleinsäuresequenzen exprimieren oder zum Beispiel durch anti-sense reprimieren muss. Er kann auch zum Beispiel künstliche Transkriptionsfaktoren vom Typ der Zinkfingerproteine verwenden (Beerli RR et al. (2000) Proc Natl Acad Sci USA 97(4):1495-500). Diese Faktoren lagern sich in den regulatorischen Bereichen der zu exprimierenden oder zu reprimierenden endogenen Gene an und bewirken, je nach Gestaltung des Faktors, eine Expression oder Repression des endogenen Gens. So kann man die gewünschten Effekte auch durch Expression eines entsprechenden Zinkfinger-Transkriptionsfaktors unter Kontrolle eines der erfindungsgemäßen Promotoren erreichen.

Die erfindungsgemäßen Expressionskassetten können ebenso zur Verminderung (Suppression) von Transkription oder/und Translation von Zielgenen durch "gene silencing" eingesetzt werden. So können die erfindungsgemäßen Expressionskassetten Nukleinsäuren exprimieren, die ein PTGS ("post transkriptional gene silencing") oder TGS ("transcriptional silencing") Effekte und so eine Verminderung der Expression endogener Gene bewirken. Besagte Verminderung kann beispielsweise durch Expression einer "anti-sense"-RNA (u.a. EP-A1 0 458 367; EP-A1 0 140 308; van der Krol AR et al. (1988) BioTechniques 6(10):658-676; de Lange P et al. (1995) Curr Top Microbiol Immunol 197:57-75) oder einer doppelsträngigen RNA, die jeweils Homologie zu dem zu vermindernden endogenen Zielgen aufweisen, erreicht werden. Auch die Expression einer entsprechenden "sense"-RNA kann mittels sogenannter Co-Suppression eine Verminderung der Expression endogener Gene bewirken (EP-A1 0 465 572). Darüberhinaus gibt es weitere Verfahren wie z.B. die Regulation der Genexpression mittels viraler Expressionssysteme ("virus induced gene silencing" VIGS; WO 98/36083, WO 99/15682). Insbesondere bevorzugt ist die Expression einer doppelsträngigen RNA zur Verminderung der Genexpression eines Zielgens. WO 99/32619 und WO 99/53050 beschreiben Verfahren zur Inhibition einzelner Zielgene unter Verwendung einer RNA mit doppelsträngiger Struktur, wobei das zielgen und die Region der RNA Duplex zumindest eine teilweise Identität aufweisen (siehe auch: Montgomery MK et al. (1998) Proc Natl Acad Sci USA 95:15502- 15507; Sharp PA (1999) Genes & Development 13(2):139-141; Fire A et al. (1998) Nature 391:806-11). Das Verfahren wird heute auch als "RNA-Interference" (RNAi) bezeichnet.

Bevorzugte Anwendungen, bei denen die Verminderung (Suppression) der Genexpression einen vorteilhaften Phänotyp bedingt, umfassen beispielhaft, aber nicht einschränkend:
1. Modifikation der Kohlenhydratzusammensetzung
   Eine Modifikation der Kohlehydratzusammensetzung kann beispielsweise erreicht werden durch Verminderung der Genexpression von Genen des Kohlenhydratstoffwechsels oder der Kohlenhydratbiosynthese, beispielsweise der Biosynthese von Amylose, Pektinen, Cellulose oder Zellwandkohlenhydraten. Dadurch kann eine Vielzahl zellulärer Prozesse (Reifung, Stärkezusammensetzung oder -gehalt etc.) in vorteilhafter Weise beeinflusst werden. Als Zielgene seien beispielhaft jedoch nicht einschränkend zu nennen Phosphorylasen, Stärkesynthetasen, Verzweigungsenzyme, Sucrose-6-phosphatsynthetasen, Sucrose-6-phosphatphosphatasen, Lipoxygenasen (Griffiths A. et al. (1999) Postharvest Biology & Technology 17(3):163-173), ADP-Glucosepyrophosphorylasen, Branching-Enzyme, Debranching-Enzyme sowie diverse Amylasen. Die entsprechenden Gene sind beschrieben (Dunwell JM (2000) J Exp Botany 51Spec No: 487-96; Brar DS et al. (1996) Biotech Genet Eng Rev 13:167-79; Kishore GM und Somerville CR (1993) Curr Opin Biotech 4(2):152-8). Vorteilhafte Gene zur Beeinflussung des Kohlenhydratstoffwechsels - insbesondere der Stärkebiosynthese - sind beschrieben in WO 92/11375, WO 92/11376, US 5,365,016 und WO 95/07355.
   In einer weiteren vorteilhaften Ausführungsform, kann eine Verschiebung des Amylose/Amylopektinverhältnisses in der Stärke durch Suppression beider Isoformen des Verzweigungsenzyms, die für die α-1,6-glykosidische Verknüpfung verantwortlich sind, bewirkt werden. Entsprechende Vorgehensweisen sind beschrieben (beispielsweise bei Schwall GP et al. (2000) Nat Biotechnol 18(5):551-554). Bevorzugt werden dazu Nukleinsäuresequenzen wie die des Starch branching enzyme II der Kartoffel (GenBank Acc.-No.: AR123356; US 6,169,226) oder dessen Homologe aus anderen Gattungen und Arten verwendet.
   Insbesondere vorteilhaft ist die Verminderung der Stärkemobilisierung und Umsetzung zu Zuckern bei niedrigen Temperaturen ("cold-sweetening") mittels Verminderung der Expression einer Glukanphosphorylase (systematischer Name: 1,4-α-D-Glukan:phosphate-α-D-glucosyltransferase; US 5,998,710).
2. Verzögerung der Fruchtreifung
   Eine verzögerte Fruchtreifung oder ein modifizierter Reifungsphänotyp (verlängerte Reifung, spätere Senescence) kann beispielsweise erreicht werden durch Verminderung der Genexpression von Genen ausgewählt aus der Gruppe bestehend aus Polygalacturonasen, Pectinesterasen, β-(1-4)Glukanasen (Cellulasen), β-Galactanasen (β-Galactosidasen), oder Gene der Ethylenbiosynthese wie 1-Aminocyclopropan-1-carboxylatsynthase, Adenosylmethioninhydrolase (SAMase), A-minocyclopropan-1-carboxylatdeaminase, Aminocyclopropan-1-carboxylatoxidas, Gene der Carotinoidbiosynthese wie z.B. Gene der Prephytoen- oder Phytoenbiosynthese beispielsweise Phytoendeaaturasen, sowie 0-Methyltransferasen, Acyl-carrier Protein (ACP), Elongationsfaktor, Auxin-induziertes Gen, Cysteine(thiol)proteinasen, Stärkephosphorylasen, Pyruvatdecarboxylasen, Chalconreduktasen, Proteinkinasen, "auxinrelated gene", Sucrosetransporter, "meristem Pattern gene". Weitere vorteilhafte Gene sind beschrieben, bespielsweise in WO 91/16440, WO 91/05865, WO 91/16426, WO 92/17596, WO 93/07275 oder WO 92/04456. Insbesondere bevorzugt ist die Verminderung der Expression der Polygalakturonase zur Verhinderung von Zellabbau und "Matschig"-werden von Pflanzen und Früchten beispielsweise von Tomaten. Bevorzugt werden dazu Nukleinsäuresequenzen wie die des Polygalakturonase-Gens der Tomate (GenBank Acc.-No.: X14074) oder dessen Homologe verwendet.
3. Verbesserter Schutz gegen abiotische Stressfaktoren (Hitze, Kälte, Trockenheit, erhöhte Feuchtigkeit, Umweltgifte, UV-Strahlung). Bevorzugt werden Gene in ihrer Expression vermindert, die an Stressreaktionen beteiligt sind.
4. Verminderung des Gehaltes von Speicherproteinen
   Die Verminderung der Genexpression von Genen kodierend für Speicherproteine (infolge SP) hat zahlreiche Vorteile, wie beispielsweise Verminderung des allergenen Potentials oder Veränderung in der Zusammensetzung oder Menge anderer Metabolite wie beispielsweise Öl- oder Stärkegehalt.
5. Erreichen einer Resistenz gegen pflanzliche Pathogene
   Eine Resistenz gegen pflanzliche Pathogene wie Arachniden, Pilze, Insekten, Nematoden, Protozoen, Viren, Bakterien und Krankheiten kann erreicht werden durch Verminderung der Genexpression von Genen, die für das Wachstum, Überleben, bestimmte Entwicklungsstufen (beispielsweise Verpuppung) oder die Vermehrung eine bestimmten Pathogens essentiell sind. Eine entsprechende Verminderung kann eine vollständige Inhibition vorgenannter Schritte aber auch eine Verzögerung derselben bewirken. Dies können pflanzliche Gene sein, die dem Pathogen beispielsweise das Eindringen ermöglichen, können aber auch pathogen-eigene Gene sein. Bevorzugt ist die transgen exprimierte Nukleinsäuresequenz (z.B. die doppelsträngige RNA) gegen Gene des Pathogens gerichtet. Als anti-pathogenes Agens kann dabei die transgen exprimierte Nukleinsäuresequenz (z.B. die doppelsträngige RNA) selber, jedoch auch die Expressionskassetten oder transgenen Organismen wirken. Die Pflanzen selber können in Form eines transgenen Organismus die Agentien beinhalten und diese - beispielsweise in Form eines Fraßgiftes - an die Pathogene weitergeben. Verschiedene essentielle Gene diverser Pathogene sind dem Fachmann bekannt (z.B. für Nematodenresistenz WO. 93/10251, WO 94/17194).
6. Am meisten bevorzugt als Pathogen sind Pilzpathogene wie Phytophthora infestans, Fusarium nivale, Fusarium graminearum, Fusarium culmorum, Fusarium oxysporum, Blumeria graminis, Magnaporthe grisea, Sclerotinia sclerotium, Septoria nodorum, Septoria tritici, Alternaria brassicae, Phoma lingam, bakterielle Pathogene wie Corynebacterium sepedonicum, Erwinia carotovora, Erwinia amylovora, Streptomyces scabies, Pseudomonas syringae pv. tabaci, Pseudomonas syringae pv. phaseolicola, Pseudomonas syringae pv. tomato, Xanthomonas campestris pv. malvacearum und Xanthomonas campestris pv. oryzae, und Nematoden wie Globodera rostochiensis, G. pallida, Heterodera schachtii, Heterodera avenae, Ditylenchus dipsaci, Anguina tritici und Meloidogyne hapla.
7. Eine Virusresistenz kann beispielsweise durch Verminderung der Expression eines viralen Hüllproteins, einer viralen Replikase, einer viralen Protease etc. erreicht werden. Zahlreiche Pflanzenviren und entsprechende Zielgene sind dem Fachmann bekannt.
8. Verminderung unerwünschter, allergener oder toxischer Pflanzeninhaltsstoffe wie z.B. Glucosinolaten oder Patatin. Entsprechende Zielgene sind beschrieben (u.a. in WO 97/16559). Die zur Verminderung von allergenen Proteinen bevorzugten Zielgene sind beispieleweise beschrieben bei Tada Y et al. (1996) FEBS Lett 391(3): 341-345 oder Nakamura R (1996) Biosci Biotechnol Biochem 60(8) :1215-1221.
9. Verzogerung von Alterserscheinungen, Entsprechende Zielgene sind u.a. Cinnamoyl-CoA, NADPH-Reduktasen oder Cinnamoylalkoholdehydrogenasen. Weitere Zielgene sind beschrieben (u.a. in WO 95/07993).
10. Verminderung der Stoßanfalligkeit von beispielsweise Kartoffeln durch Verminderung beispielsweise der Polyphenoloxidase (WO 94/03607) etc.
11. Erhöhung des Methioningehaltes durch Verminderung der Threoninbiosynthese, beispielsweise durch Verminderung der Expression der Threoninsynthase (Zeh M et al. (2001) Plant Physiol 127(3) : 792-802).

Eine "antisense" Nukleinsäure meint zunächst eine Nukleinsäuresequenz die ganz oder teilsweise zu zumindest einem Teil des "sense"-Stranges besagten Zielproteins komplementär ist. Dem Fachmann ist bekannt, dass er alternativ die cDNA oder das korrespondierende Gen als Ausgangsmatrize für entsprechende antisense-Konstrukte verwenden kann. Bevorzugt ist die "antisense" Nukleinsäure komplementär zu dem kodierenden Bereich des Zielproteins oder einem Teil desselben. Die "antisense" Nukleinsäure kann aber auch zu der nicht-kodierenden Region oder einem Teil derselben komplementär sein. Ausgehend von der Sequenzinformation zu einem Zielprotein, kann eine antisense Nukleinsäure unter Berücksichtigung der Basenpaarregeln von Watson und Crick in der dem Fachmann geläufigen Weise entworfen werden. Eine antisense Nukleinsäure kann komplementar zu der gesamten oder einem Teil der Nukleinsäuresequenz eines Zielproteins sein. In einer bevorzugten Ausführungsform ist die antisense Nukleinsäure ein Oligonukleotid mit einer Länge von zum Beispiel 25, 30, 35, 40, 45 oder 50 Nukleotiden.

Vorteilhaft kann die antisense-Strategie mit einem Ribozym-Verfahren gekoppelt werden. Ribozyme sind katalytisch aktive RNA Sequenzen, die gekoppelt an die antisense Sequenzen, die Zielsequenzen katalytisch spalten (Tanner NK (1999) FEMS Microbiol Rev 23(3):257-75). Dies kann die Effizienz einer anti-sense Strategie erhöhen. Die Expression von Ribozymen zur Verminderung bestimmter Proteine ist dem Fachmann bekannt und beispielsweise beschrieben in EP-A1 0 291 533, EP-A1 0 321 201 und EP A1 0 360 257. Geeignete Zielsequenzen und Ribozyme können zum Beispiel wie bei Steinecke (Ribozymes, Methods in Cell Biology 50, Galbraith et al eds Academic Press, Inc. (1995), 449-460) beschrieben, durch Sekundärstrukturberechnungen von Ribozym- und Ziel-RNA sowie durch deren Interaktion bestimmt werden (Bayley CC et al.(1992) Plant Mol Biol 18(2):353-361; Lloyd AM and Davis RW et al. (1994) Mol Gen Genet 242(6):653-657). Beispielhaft sind "hammerhead"-Ribozyme zu nennen (Haselhoff and Gerlach (1988) Nature 334:585-591). Bevorzugte Ribozyme basieren auf Derivaten der Tetrahymena L-19 IVS RNA (US 4,987,071; US 5,116,742). Weitere Ribozyme mit Selektivität für eine L119 mRNA können selektioniert werden (Bartel D und Szostak JW (1993) Science 261:1411-1418).

Ebenso umfasst ist die Verwendung der oben beschriebenen Sequenzen in sense-Orientierung, was wie dem Fachmann geläufig ist, zu einer Kosuppression führen kann. Die Expression von sense-RNA zu einem endogenen Gen kann die Expression desselben vermindern oder ausschalten, ähnlich wie es für antisense Ansätze beschrieben wurde (Goring et al. (1991) Proc. Natl Acad Sci USA, 88:1770-1774; Smith et al. (1990) Mol Gen Genet 224:447-481; Napoli et al. (1990) Plant Cell 2:279-289; Van der Krol et al. (1990) Plant Cell 2:291-299). Dabei kann das eingeführte Konstrukt das zu vermindernde Gen ganz oder nur teilweise repräsentieren. Die Möglichkeit zur Translation ist nicht erforderlich.

Ganz besonders bevorzugt ist auch die Verwendung von Verfahren wie der Genregulation mittels doppelsträngiger RNA ("double-stranded RNA interference"). Entsprechende Verfahren sind dem Fachmann bekannt und im Detail beschrieben (z.B. Matzke MA et al. (2000) Plant Mol Biol 43:401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Hier wird durch gleichzeitige Einbringung von Strang- und Gegenstrang eine hocheffiziente Unterdrückung nativer Gene bewirkt.

Die erfindungsgemäßen Expressionskassetten und die von ihnen abgeleiteten Expressionsvektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten oder von diesen abgeleitete Expressionsvektoren oder Organismen haben. Beispielhaft aber nicht einschränkend seien zu nennen:

### 1. Selektionsmarker

Der Begriff "Selektionsmarker" umfasst sowohl positive Selektionsmarker, die eine Resistenz gegen ein Antibiotikum, Herbizid oder anderes Biozid verleihen, als auch negative Selektionsmarker, die eine Sensitivität gegen eben diese verleihen, als auch Marker die dem transformierten Organismus einen Wachstumsvorteil gewähren (beispielsweise durch Expression von Schlüsselgenen der Cytokinbiosynthese; Ebinuma H et al. (2000) Proc Natl Acad Sci USA 94:2117-2121). Bei der positiven Selektion gedeihen nur die Organismen, die den entsprechenden Selektionsmarker exprimieren, während bei der negativen Selektion eben diese eingehen. Bei der Herstellung transgener Pflanzen ist die Verwendung eines positiven Selektionsmarkers bevorzugt. Bevorzugt ist ferner die Verwendung von Selektionsmarkern, die Wachstumsvorteile verleihen. Negative Selektionsmarker können vorteilhaft verwendet werden, wenn es darum geht, bestimmte Gene oder Genomabschnitte aus einem Organismus zu entfernen (beispielsweise im Rahmen eines Kreuzungsprozesses).

### i) Positive Selektionsmarker:

Der mit der Expressionskassette eingebrachte selektionierbare Marker verleiht den erfolgreich transformierten Zellen eine Resistenz gegen ein Biozid, zum Beispiel ein Herbizid (wie Phosphinothricin, Glyphosat oder Bromoxynil), einen Metabolismusinhibitor (wie 2-Desoxyglucose-6-phosphat; WO 98/45456) oder ein Antibiotikum (wie z.B. Tetracycline, Ampicillin, Kanamycin, G 418, Neomycin, Bleomycin oder Hygromycin). Der Selektionsmarker erlaubt die Selektion der transformierten Zellen von untransformierten (McCormick et al.(1986) Plant Cell Reports 5: 81-84). Besonders bevorzugte Selektionsmarker sind solche, die eine Resistenz gegen Herbizide verleihen. Beispielhaft als Selektionsmarker seien zu nennen:
- DNA Sequenzen, die für Phosphinothricinacetyltransferasen (PAT) kodieren (auch Bialophos^{®} -Resistenzgen (bar) genannt), welche die freie Aminogruppe des Glutaminsynthaseinhibitors Phosphinothricin (PPT) acetylieren und damit eine Detoxifizierung des PPT erreichen (de Block et al. (1987) EMBO J 6:2513-2518; Vickers JE et al. (1996) Plant Mol Biol Reporter 14:363-368; Thompson CJ et al. (1987) EMBO J 6:2519-2523). Das bar/PAT Gen kann beispielsweise aus Streptomyces hygroscopicus oder S. viridochromogenes isoliert werden. Entsprechende Sequenzen sind dem Fachmann bekannt (Streptomyces hygroscopicus GenBank Acc.-No.: X17220 und X05822; Streptomyces viridochromogenes GenBank Acc.-No.: M22827 und X65195; US.5,489,520). Ferner sind synthetische Gene für die Expression in Plastiden beschrieben (Genbank Acc.-No.: AJ028212).
- 5-Enolpyruvylshikimat-3-phosphatsynthasegene (EPSP Synthasegene), die eine Resistenz gegen Glyphosat^{®} (N-(phosphonomethyl)glycin) verleihen. Das unselektive Herbizid Glyphosat hat die 5-Enolpyruvyl-3-phosphoshikimatsynthase (EPSPS) als molekulares Target. Diese hat eine Schlüsselfunktion in der Biosynthese aromatischer Aminosäuren in Pflanzen (Steinrucken HC et al. (1980) Biochem Biophys Res Commun 94:1207-1212; Levin JG und Sprinson DB (1964) J Biol Chem 239:1142-1150; Cole DJ (1985) Mode of action of glyphosate; A literature analysis, p. 48-74. In: Grossbard E und Atkinson D (eds.) The herbicide glyphosate. Buttersworths, Boston). Glyphosat-tolerante EPSPS Varianten werden bevorzugt als Selektionsmarker verwendet (Padgette SR et al. (1996). New weed control opportunities: development of soybeans with a Roundup Ready™ gene. In: Herbicide Resistant Crops (Duke SO, ed.), pp. 53-84. CRC Press, Boca Raton, FL; Saroha MK und Malik VS (1998) J Plant Biochemistry and Biotechnology 7:65-72). Das EPSPS Gen des Agrobakterium sp. Stamm CP4 hat eine natürliche Toleranz gegen Glyphosat, die auf entsprechende transgene Pflanzen transferiert werden kann. Das CP4 EPSPS Gen wurde aus Agrobakterium sp. Stamm CP4 kloniert (Padgette SR et al. (1995) Crop Science 35(5):1451-1461). 5-Enolpyrvylshikimate-3-phosphate-synthasen, die Glyphosat-tolerant sind, wie beispielsweise beschrieben in US 5,510,471; US 5,776,760; US 5,864,425; US 5,633,435; US 5,627;061; US 5,463,175; EP 0 218 571, sind bevorzugt, wobei die jeweils in den Patenten beschriebenen Sequenzen auch in der GenBank hinterlegt sind. Weitere Sequenzen sind beschrieben unter GenBank Accession X63374. Ferner ist das aroA Gen bevorzugt (GenBank Acc.-No.: M10947).
- das für das Glyphosat^{®} degradierende Enzyme kodierende gox Gen (Glyphosatoxidoreduktase). GOX (beispielsweise die Glyphosatoxidoreductase aus Achromobacter sp.) katalysiert die Spaltung einer C-N Windung im Glyphosat, welches so zu Aminomethylphosphonsäure (AMPA) und Glyoxylat umgesetzt wird. GOX kann dadurch eine Resistenz gegen Glyphosat vermitteln (Padgette SR et al. (1996) J Nutr 126(3):702-16; Shah D et al. (1986) Science 233:478-481).
- das deh Gen (kodierend für eine Dalapon^{®} inaktivierende Dehalogenase; WO 99/27116; GenBank Acc.-NO.: AX022822, AX022820)
- bxn Gene, die für Bromoxynil^{®} degradierende Nitrilaseenzyme kodieren. Beispielsweise die Nitrilase aus Klebsiella ozanenae. Sequenzen sind in der GenBank beispielsweise unter den Genbank Acc.-No: E01313 und J03196 zu finden.
- Neomycinphoaphotransferasen (npt) verleihen eine Resistenz gegen Antibiotika (Aminoglykoside) wie Neomycin, G418, Hygromycin, Paromomycin oder Kanamycin, indem sie durch eine Phosphorylierungsreaktion deren inhibierende Wirkung reduzieren. Besonders bevorzugt ist das nptII Gen (Genbank Acc.-No.:
AF080390; AF080389). Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden (Genbank Acc.-No.: AF234316 pCAMBIA-2301; AF234315 pCAMBIA-2300, AF234314 pCAMBIA-2201). Das NPTII Gen kodiert für eine Aminoglycosid-3'O-phosphotransferase aus E.coli, Tn5 (GenBank Acc.-No: U00004 Position 1401-2300; Beck et al. (1982) Gene 19 327-336).
- das DOG^{R}1-Gen wurde aus der Hefe Saccharomyces cerevisiae isoliert (EP 0 807 836) und kodiert für eine 2-Desoxyglukose-6-phosphatphosphatase, die eine Resistenz gegenüber 2-DOG verleiht (Randez-Gil et al. (1995) Yeast 11:1233-1240; Sanz et al. (1994) Yeast 10:1195-1202; GenBank Acc.-No.: NC001140 Position 194799-194056).
- Sulfonylharnstoff- und Imidazolinon inaktivierende Acetolactatsynthasen, die eine Resistenz gegen Imidazolinon/Sulfonylharnstoff-Herbizide verleihen. Beispielhaft seien für Imidazolinon-Herbizide die Wirkstoffe Imazamethabenzmethyl, Imazzamox, Imazapyr, Imazaquin, Imazethapyr zu nennen. Für Sulfonylharnstoff-Herbizide seien beispielhaft Amidosulforon, Azimsulfuron, Chlorimuronethyl, Chlorsulfuron, Cinosulfuron, Imazosulforon, Oxasulforon, Prosulforon, Rimsulforon, Sulfosulforon zu nennen. Dem Fachmann sind zahlreiche weitere Wirkstoffe der genannten Klassen bekannt. Geeignet sind beispielsweise die unter der GenBank Acc-No.: X51514 hinterlegte Sequenz für das Arabidopsis thaliana Csr 1.2 Gen (EC 4.1.3.18) (Sathasivan K et al. (1990) Nucleic Acids Res. 18(8):2188). Acetolactatesynthasen die eine Resistenz gegen Imidazolinon-Herbizide verleihen sind ferner beschrieben unter den GenBank Acc.-No.: AB049823, AF094326, X07645, X07644 , A19547, A19S46, A19545, 105376 (EP 0 257 993), I05373 (EP 0 257 993), AL133315.
- Hygromycinphosphotransferasen (z.B. GenBank Acc.-No.: X74325), die eine Resistenz gegen das Antibiotikum Hygromycin verleihen. Das Gen ist Bestandteil zahlreicher Expressionsvektoren und kann unter Verwendung von dem Fachmann geläufigen Verfahren (wie beispielsweise Polymerasekettenreaktion) aus diesen isoliert werden (GenBank Acc.-No.: AF294981 pINDEX4; AF234301 pCAMBIA-1380; AF234300 pCAMBIA-1304; AF234299 pCAMBIA-1303; AF234298 pCAMBIA-1302; AF354046 pCAMBIA-1305.; AF354045 pCAMBIA-1305.1)
- Resistenzgene gegen
   a) Chloramphenicol (Chloramphenicolacetyltransferase),
   b) Tetracyclin, verschiedene Resistenzgene sind beschrieben z.B. GenBank Acc.-No.: X65876, X51366. zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden
   c) Streptomycin, verschiedene Resistenzgene sind beschrieben z.B. mit der GenBank Acc.-No.: AJ278607.
   d) Zeocin, das entsprechende Resistenzgen ist Bestandteil zahlreicher Klonierungsvektoren (z.B. GenBank Acc.-No.: L36849 Cloning vector pZEO) und kann unter Verwendung von dem Fachmann geläufigen Verfahren (wie beispielsweise Polymerasekettenreaktion) aus diesen isoliert werden.
   e) Ampicillin (β-Lactamase Gen; Datta N, Richmond MH.(1966) Biochem J 98(1):204-9: Heffron F et al (1975) J. Bacteriol 122: 250-256; das Amp Gen wurde zuerst zur Herstellung des E. coli Vektors pBR322 kloniert; Bolivar F et al. (1977) Gene 2:95-114). Die Sequenz ist Bestandteil zahlreicher Klonierungsvektoren und kann unter Verwendung von dem Fachmann geläufigen Verfahren (wie beispielsweise Polymerasekettenreaktion) aus diesen isoliert werden.
- Gene wie die Isopentenyltransferase aus Agrobakterium tumefaciens (strain:PO22) (Genbank Acc.-No.: AB025109). Das ipt Gen ist ein Schlüsselenzym der Cytokin-Biosynthese. Seine Überexpression erleichtert die Regeneration von Pflanzen (z.B. Selektion auf Cytokin-freiem Medium). Das Verfahren zur Nutzung des ipt Gens ist beschrieben (Ebinuma H et al. (2000) Proc Natl Acad Sci USA 94:2117-2121; Ebinuma H et al. (2000) Selection of Marker-free transgenic plants using the oncogenes (ipt, rol A, B, C) of Agrobakterium as selectable markers, In Molecular Biology of Woody Plants. Kluwer Academic Publishers).
- Verschiedene weitere positive Selektionsmarker, die den transformierten Pflanzen einen Wachstumsvorteil gegenüber nichttransformierten verleihen, sowie Verfahren zu ihrer Verwendung sind u.a. beschrieben in EP-A 0 601 092. Beispielhaft sind zu nennen β-Glucuronidase (in Verbindung mit z.B. Cytokininglucuronid), Mannose-6-phosphat-Isomerase (in Verbindung mit Mannose), UDP-Galaktose-4-Epimerase (in Verbindung mit z.B. Galactose), wobei Mannose-6-phosphat-Isomerase in Verbindung mit Mannose besonders bevorzugt ist.

### ii) Negative Selektionsmarker

Negative Selektionsmarker ermöglichen beispielsweise die Selektion von Organismen mit erfolgreich deletierten Sequenzen, die das Markergen umfassen (Koprek T et al. (1999) The Plant Journal 19(6):719-726). Bei der negativen Selektion wird beispielsweise durch den in die Pflanze eingebrachten negativen Selektionsmarker eine Verbindung, die ansonsten für die Pflanze keine nachteilige Wirkung hat, in eine Verbindung mit nachteiliger Wirkung umgesetzt. Ferner sind Gene geeignet, die per se eine nachteilige Wirkung haben. Als negative Selektionsmarker seien beispielhaft jedoch nicht einschränkend zu nennen TK thymidine kinase (TK), Diphtheria Toxin A Fragment (DT-A), das codA Genprodukt kodierend für eine Cytosindeaminase (Gleave AP et al. (1999) Plant Mol Biol. 40(2):223-35; Perera RJ et al. (1993) Plant Mol Biol 23(4): 793-799; Stougaard J; (1993) Plant J 3:755-761), das Cytochrom P450 Gen (Koprek et al. (1999) Plant J 16:719-726), Gene kodierend für eine Haloalkandehalogenase (Naested H (1999) Plant J. 18:571-576), das iaaH Gen (Sundaresan V et al. (1995) Genes & Development 9:1797-1810) oder das tms2 Gen (Fedoroff NV & Smith DL (1993) Plant J 3:273-289).

### 2) Reportergene

Reportergene kodieren für leicht quantifizierbare Proteine und gewährleisten über Eigenfarbe oder Enzymaktivität eine Bewertung der Transformationseffizienz, des Expressionsortes oder - zeitpunktes (siehe auch Schenborn E, Groskreutz D. Mol Biotechnol. 1999; 13(1):29-44). Beispielhaft seien zu nennen:
- "green fluorescence protein" (GFP) (Chui WL et al. (1996), Curr Biol 6:325-330; Leffel SM et al. (1997) Biotechniques 23(5):912-8; Sheen et al.(1995) Plant J 8(5):777-784; Haseloff et al.(1997) Proc Natl Acad Sci USA 94(6):2122-2127; Reichel et al. (1996) Proc Natl Acad Sci USA 93(12):5888-5893; Tian et al. (1997) Plant Cell Rep 16:267-271; WO 97/41228).
- Chloxamphenicoltransferase (Fromm et al. (1985) Proc Natl Acad Sci USA 82:5824-5828),
- Luziferase (Millar et al. (1992) Plant Mol Biol Rep 10:324-414; Ow et al. (1986) Science 234:856-859); erlaubt Bioluminescenzdetektion.
- β-Galactosidase, kodiert für ein Enzym für das verschiedenen chromogene Substrate zur Verfügung stehen.
- β-Glucuronidase (GUS) (Jefferson et al. (1987) EMBO J 6:3901-3907) oder das uidA Gen, das ein Enzym für verschiedene chromogene Substrate kodiert.
- R-Locus Genprodukt:Protein, das die Produktion von Anthocyaninpigmenten (rote Färbung) in pflanzlichen Gewebe reguliert und so eine direkte Analyse der Promotoraktivität ohne Zugabe zusätzlicher Hilfsstoffe oder chromogener Substrate ermöglicht (Dellaporta et al. (1988) In: Chromosome Structure and Function: Impact of New Concepts, 18th Stadler Genetics Symposium, 11:263-282).
- Tyrosinase (Katz et al. (1983) J Gen Microbiol 129:2703-2714) ein Enzym, das Tyrosin zu DOPA und Dopaquinon oxidiert, die infolge das leicht nachweisbare Melanin bilden.
- Aequorin (Prasher et al. (1985) Biochem Biophys Res Commun 126(3):1259-1268), kann in der Calcium-sensitiven Bioluminescenzdetektion verwendet werden.

### 3) Replikationsursprünge

Replikationsursprünge gewährleisten die Vermehrung der erfindungsgemäßen Expressionskassetten oder Expressionsvektoren in zum Beispiel E. coli oder Agrobakterien. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989). Beispielshaft für in Agrobakterium funktionelle Replikationsursprünge seien pRK2, pRi, PVS1 oder pSA zu nennen.

### 4) Bordersequenzen

"Bordersequenzen" (wie z.B, die rechte oder linke Begrenzung der T-DNA) ermöglichen einen Agrobakterien-vermittelten Transfer in Pflanzenzellen für die Übertragung und Integration ins Pflanzengenom.

### 5) Multiple Klonierungsregionen (MCS) erlauben und erleichtern die Insertion eines oder mehrerer Nukleinsäuresequenzen,

Erfindungsgemäß sind ferner Expressionsvektoren, die die oben beschriebenen Expressionskassetten enthalten. Vektoren meint allgemein replikationsfähige Strukturen, die bevorzugt wirtspezifisch sind, und die Aufnahme von Nukleinsäuresequenzen und deren Transfer in andere Zellen ermöglichen. Vektoren können beispielhaft Plasmide, Cosmide, Phagen, Viren oder auch Agrobakterien sein. Im Rahmen der Pflanzenbiotechnologie insbesondere geeignete Vektoren sind unten beschrieben.

Ein anderer Gegenstand der Erfindung betrifft transgene Organismen, transformiert mit wenigstens einer erfindungsgemäßen Expressionskassette oder einem erfindungsgemäßen Expressionsvektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen.

Erfindungsgemäß werden unter Organismus, Ausgangs- oder Wirtsorganismen Mikroorganismen oder Pflanzen enthaltend ein Kohlenhydrat-speicherndes Sink-Gewebe verstanden. Bevorzugte Mikroorganismen sind Bakterien, Hefen, Algen oder Pilze.

Bevorzugte Bakterien sind Bakterien der Gattung Escherichia, Erwinia, Agrobakterium, Flavobacterium, Alcaligenes oder Cyanobakterien zum Beispiel der Gattung Synechocystis.

Bevorzugt sind vor allem Mikroorganismen, welche zur Infektion von Pflanzen und damit zur Übertragung der erfindungsgemäßen Kassetten befähigt sind. Bevorzugte Mikroorganismen sind solche aus der Gattung Agrobakterium und insbesondere der Art Agrobakterium tumefaciens.

Bevorzugte Hefen sind Candida, Saccharomyces, Hansenula oder Pichia.

Bevorzugte Pilze sind Aspergillus, Trichodermä, Ashbya, Neurospora, Fusarium, Beauveria oder weitere in Indian Chem Engr. Section B. Vol 37, No 1,2 (1995) auf Seite 15, Tabelle 6 beschriebene Pilze.

In einer Ausführungsform sind Wirts- oder Ausgangsorganismen pflanzliche Organismen. Pflanzliche Organismen meint allgemein all solche zur Photosynthese befähigten Organismen.

Eingeschlossen sind als pflanzliche Organismen im Rahmen der Erfindung alle Gattungen und Arten höherer und niedrigerer Pflanzen des Pflanzenreiches. Eingeschlossen sind ferner die reifen Pflanzen, Saatgut, Knollen, Rüben, Früchte, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungestadium jenseits des Keimlings. Keimling meint eine junge, unreife Pflanze in einem frühen Entwicklungsstadium.

Einjährige, mehrjährige, monocotyledone und dicotyledone Pflanzen sind bevorzuge Wirtsorganismen für die Herstellung transgener Pflanzen. Die Expression von Genen ist ferner vorteilhaft bei allen Schmuckpflanzen, Nutz- oder Zierbäumen, Blumen, Schnittblumen, Sträuchern oder Rasen. Beispielhaft aber nicht einschränkend seien zu nennen Angiospermen, Bryophyten wie zum Beispiel Hepaticae (Leberblümchen) und Musci (Moose); Pteridophyten wie Farne, Schachtelhalm und Lycopoden; Gymnospermen wie Koniferen, Cycaden, Ginkgo und Gnetalen; Algen wie Chlorophyceae, Phaeophpyceae, Rhodophyceae, Myxophyceae, Xanthophyceae, Bacillariophyceae (Diatomeen) und Euglenophyceae.

Bevorzugt sind Pflanzen nachfolgender Pflanzenfamilien: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae, Umbelliferae.

Bevorzugte monokotyle Pflanzen sind insbesondere ausgewählt aus den monokotylen Kulturpflanzen, wie zum Beispiel der Familie der Gramineae wie Reis, Mais, Weizen oder andere Getreidearten wie Gerste, Hirse, Roggen, Triticale oder Hafer sowie dem Zuckerrohr sowie alle Arten von Gräsern.

Bevorzugte dikotyle Pflanzen sind insbesondere ausgewählt aus den dikotylen Kulturpflanzen, wie zum Beispiel
- Asteraceae wie Sonnenblume, Tagetes oder Calendula und andere mehr,
- Compositae, besonders die Gattung Lactuca, ganz besonders die Art sativa (Salat) und andere mehr,
- Cruciferae, besonders die Gattung Brassica, ganz besonders die Arten napus (Raps), campestris (Rübe), oleracea cv Tastie (Kohl), oleracea cv Snowball Y (Blumenkohl) und oleracea cv Emperor (Broccoli) und weitere Kohlarten; und der Gattung Arabidopsis, ganz besonders die Art thaliana sowie Kresse oder Canola und andere mehr,
- Cucurbitaceae wie Melone, Kürbis oder Zucchini und andere mehr,
- Leguminosae besonders die Gattung Glycine, ganz besonders die Art max (Sojabohne) Soja sowie Alfalfa, Erbse, Bohnengewächsen oder Erdnuss und andere mehr
- Rubiaceae, bevorzugt der Unterklasse Lamiidae wie beispielsweise Coffea arabica oder Coffea liberica (Kaffestrauch) und andere mehr,
- Solanaceae besonders die Gattung Lycopersicon, ganz besonders die Art esculentum (Tomate), die Gattung Solanum, ganz besonders die Art tuberosum (Kartoffel) und melongena (Aubergine) und die Gattung Capsicum, ganz besonders die Art annum (Pfeffer), sowie Tabak und andere mehr,
- Sterculiaceae, bevorzugt der Unterklasse Dilleniidae wie beispielsweise Theobroma cacao (Kakaostrauch) und andere mehr,
- Theaceae, bevorzugt der Unterklasse Dilleniidae wie beispielsweise Camellia sinensis oder Thea sinensis (Teestrauch) und andere mehr,
- Umbelliferae, besonders die Gattung Daucus (ganz besonders die Art carota (Karotte) und Apium (ganz besonders die Art graveolens dulce (Selarie) und andere mehr;
- Chenopodiaceae, bevorzugt die Gattung Beta vulgaris, insbesondere die Art Beta vulgaris ssp. vulgaris var. altissima L. (Zuckerrübe) und andere mehr;
sowie Lein, Baumwolle, Hanf, Flachs, Gurke, Spinat, Möhre, Zuckerrübe und den verschiedenen Baum-, Nuss- und Weinarten, insbesondere Banane und Kiwi.

Pflanzliche Organismen im Sinne der Erfindung sind weiterhin weitere photosynthetisch aktive befähigte Organismen, wie zum Beispiel Algen, sowie Moose. Bevorzugte Algen sind Grünalgen, wie beispielsweise Algen der Gattung Haematococcus, Phaedactylum tricornatum, Volvox oder Dunaliella.

Am meisten bevorzugt sind Pflanzen der Familie Solanaceae besonders die Gattung Lycopersicon, ganz besonders die Art esculentum (Tomate), die Gattung Solanum, ganz besonders die Art tuberosum (Kartoffel) und melongena (Aubergine), der Familie Chenopodiaceae insbesondere die Gattung Beta vulgaris, insbesondere die Art Beta vulgaris ssp. vulgaris var. altissima L. (Zuckerrübe) und andere mehr, die Familie Leguminosae besonders die Gattung Glycine, ganz besonders die Art max (Sojabohne) sowie Alfalfa, Erbse, Bohnengewächsen besonders die Gattung Vicia oder Erdnuss und andere mehr und andere Pflanzen mit stärkehaltigen Samen, Knollen, Rüben, Früchten oder Geweben. Aus diesen wiederum bevorzugt sind Tomate, Kartoffel, Aubergine, Sojabohne, Alfalfa, Erbse, Ackerbohne, Futterrrübe, Zuckerrübe und Erdnuss.

Die Herstellung eines transformierten Organismus oder einer transformierten Zelle erfordert, dass die entsprechende DNA in die entsprechende Wirtszelle eingebracht wird. Für diesen Vorgang, der als Transformation bezeichnet wird, steht eine Vielzahl von Methoden zur Verfügung (siehe auch Keown et al. 1990 Methods in Enzymology 185:527-537). So kann die DNA beispielhaft direkt durch Mikroinjektion oder durch Bombardierung mit DNAbeschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden.

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte particle bombardment Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobakterium tumefaciens oder Agrobakterium rhizogenes durchgeführt werden. Diese Stämme enthalten ein Plasmid (Ti bzw. Ri Plasmid), von dem ein Teil (die sogenannte "T-DNA") auf die Pflanze nach Agrobakterium-Infektion übertragen und in das Genom der Pflanzenzelle integriert wird. Die Agrobakterium-vermittelte Transformation ist am besten für dicotyledone Pflanzenzellen geeignet, wohingegen die direkten Transformationstechniken sich für jeden Zelltyp eignen.

Die Einführung einer erfindungsgemäßen Expressionskassette in Zellen, bevorzugt in pflanzliche Zellen, kann vorteilhaft unter Verwendung von Vektoren realisiert werden.

In einer vorteilhaften Ausführungsform wird die Einführung der Expressionskassette mittels Plasmidvektoren realisiert. Bevorzugt sind solche Expressionsvektoren, die eine stabile Integration der Expressionskassette in das Wirtsgenom ermöglichen. Wirtsgenom meint in diesem Zusammenhang die gesamte Erbinformation des Wirts und umfasst beispielhaft sowohl die chromosomale DNA des Zellkerns, als auch die DNA der Plastiden und Mitochondrien. Bevorzugt erfolgt die Insertion jedoch in die chromosomale DNA des Zellkerns.

Im Falle von Injektion oder Elektroporation von DNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist es erforderlich, dass sich auf dem Plasmid ein zusätzliches selektionierbares Markergen befindet.

Transformationstechniken sind für verschiedene monokotyle und dikotyle pflanzliche Organismen beschrieben. Ferner stehen verschiedene mögliche Plasmidvektoren für die Einführung fremder Gene in Pflanzen zur Verfügung, die in der Regel einen Replikationsursprung für eine Vermehrung in E.coli und ein Markergen für eine Selektion transformierter Bakterien enthalten. Beispiele sind pBR322, pUC Reihe, M13mp Reihe, pACYC184 etc.

Die Expressionskassette kann in den Vektor über eine geeignete Restriktionsschnittstelle eingeführt werden. Das entstandene Plasmid wird zunächst in E. coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Transformierte Zellen d.h. solche, die die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide zu verleihen vermag. Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind das bar Gen, dass Resistenz gegen das Herbizid Phosphinothricin verleiht (Rathore KS et al., Plant Mol Biol. 1993 Mar; 21(5):871-884), das nptII Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht.

Je nach Methode der DNA-Einführung können weitere Gene auf dem Vektorplasmid erforderlich sein. Werden Agrobakterien verwendet, so ist die Expressionskassette in spezielle Plasmide zu integrieren, entweder in einen Zwischenvektor (englisch: shuttle or intermediate vector) oder in einen binären Vektor. Wenn zum Beispiel ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden. Bevorzugt werden binäre Vektoren verwendet. Binäre Vektoren können sowohl in E.coli als auch in Agrobakterium replizieren. Sie enthalten in der Regel ein Selektionsmarkergen und einen Linker oder Polylinker flankiert von der rechten und linken T-DNA Begrenzungssequenz. Sie können direkt in Agrobakterium transformiert werden (Holsters et al., Mol. Gen. Genet. 163 (1978), 181-187). Das Selektionsmarkergen erlaubt eine Selektion transformierter Agrobakteria und ist zum Beispiel das nptII Gen, das eine Resistenz gegen Kanamycin verleiht. Das in diesem Fall als Wirtsorganismus fungierende Agrobakterium sollte bereits ein Plasmid mit der vir-Region enthalten. Diese ist für die Übertragung der T-DNA auf die pflanzliche Zelle erforderlich. Ein so transformiertes Agrobakterium kann zur Transformation pflanzlicher Zellen verwendet werden.

Die Verwendung von T-DNA zur Transformation pflanzlicher Zellen ist intensiv untersucht und beschrieben (B. Jenes et al., Techniques for Gene Transfer, in: Transgenic Plants, Vol. 1, Engineering and Utilization, herausgegeben von Kung SD und Wu R, Academic Press (1993), S.128-143 sowie in Potrykus (1991) Annu Rev Plant Physiol Plant Molec Biol 42:205-225; EP 120516; Hoekema, In: The Binary Plant Vector System, Offsetdrukkerij Kanters B.V., Alblasserdam, Chapter V; Fraley et al., Crit. Rev. Plant. Sci., 4:1-46 and An et al. (1985) EMBO J 4:277-287). Verschiedene binäre Vektoren sind bekannt und teilweise kommerziell erhältlich wie zum Beispiel pBIN19 (Bevan et al. (1984) Nucl Acids Res 12:8711f.; Clontech Laboratories, Inc. USA) oder pSUN Derivate (SunGene GmbH & Co.KGaA; WO 02/00900). In diese binären Vektoren kann die erfindunsggemäße Expressionskassette insertiert und - wie im folgenden beschrieben - in das pflanzliche genom integriert werden,

Für den Transfer der DNA in die pflanzliche Zelle werden pflanzliche Explantate mit Agrobakterium tumefaciens oder Agrobakterium rhizogenes kokultiviert. Ausgehend von infiziertem Pflanzenmaterial (z.B. Blatt-, Wurzel- oder Stengelteile, aber auch Protoplasten oder Suspensionen von Pflanzenzellen) können ganze Pflanzen unter Verwendung eines geeigneten Mediums, dass zum Beispiel Antibiotika oder Biozide zur Selektion transformierter Zellen enthalten kann, regeneriert werden. Die erhaltenen Pflanzen können dann auf die Präsenz der eingeführten DNA, hier der erfindungsgemäßen Expressionskassette, durchmustert werden. Sobald die DNA in das Wirtsgenom integriert ist, ist der entsprechende Genotyp in der Regel stabil und die entsprechende Insertion wird auch in den Nachfolgegenerationen wiedergefunden. In der Regel enthält die integrierte Expressionskassette einen Selektionsmarker, der der transformierten Pflanze eine Resistenz gegen ein Biozid (zum Beispiel ein Herbizid), einen Metabolismusinhibitor wie 2-DOG oder ein Antibiotikum wie Kanamycin, G 418, Bleomycin, Hygromycin oder Phosphinothricin etc. verleiht. Der Selektionsmarker erlaubt die Selektion von transformierten Zellen von untransformierten (McCormick et al. (1986) Plant Cell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Die Integration der T-DNA kann z.B. durch die Wirksamkeit der Expression der transgen exprimierten Nukleinsäuren oder des Selektionsmarkers beispielsweise in vitro durch Sprossmeristemvermehrung unter Verwendung einer der oben beschriebenen Selektionsmethoden ermittelt werden.

Erfindungsgemäß sind ferner Zellen, Zellkulturen, Teile - wie zum Beispiel bei transgenen pflanzlichen Organismen wurzeln, Blätter etc.-, und transgenes Vermehrungsgut wie Saaten, Knollen, Rüben oder Früchte, enthaltend eine erfindungsgemäße Expressionskassette oder einen erfindungsgemäßen Expressionsvektor.

Von Menschen und Tieren verzehrbare erfindungsgemäße, genetisch veränderte Pflanzen können auch beispielsweise direkt oder nach an sich bekannter Aufbereitung als Nahrungsmittel oder Futtermittel verwendet werden.

Ein weiterer Gegenstand der Erfindung betrifft die Verwendung der oben beschriebenen erfindungsgemäßen, transgenen Organismen und der von ihnen abgeleitete Zellen, Zellkulturen, Teile - wie zum Beispiel bei transgenen pflanzlichen Organismen Wurzeln, Blätter etc.- , und transgenes Vermehrungsgut wie Saaten, Knollen, Rüben oder Früchte, zur Herstellung von Nahrungs- oder Futtermitteln, Pharmazeutika oder Feinchemikalien mittels Pflanzen enthaltend ein Kohlenhydrat-speicherndes Sink-Gewebe.

Bevorzugt ist ferner ein Verfahren zur rekombinanten Herstellung von Pharmazeutika oder Feinchemikalien in Wirtsorganismen wobei ein Wirtsorganismus mit einer der oben beschriebenen Expressionskassetten oder Expressionsvektoren transformiert wird und diese Expressionskassette ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mittels der dem Fachmann bekannten Verfahren. Die Produktion von Pharmazeutika, wie zum Beispiel Antikörpern oder Vakkzinen ist beschrieben bei Hood EE Jilka JM (1999) Curr Opin Biotechnol 10(4):382-6; Ma JK Vine ND (1999) Curr Top Microbiol Immunol 236:275-92.

### Sequenzen

| | |
|---|---|
| 1. SEQ ID NO: 1 | Promotor des Vicia faba Phol-Gens |
| | |
| 2. SEQ ID NO: 2 | Promotor und 5'-untranslatierte Region des Vicia faba Phol-Gens |
| | |
| 3. SEQ ID NO: 3 | Promotor und 5'-untranslatierte Region des Vicia faba Phol-Gens sowie Sequenz kodierend für das Transitpeptid des Vicia faba Phol-Proteins. |
| | |
| 4. SEQ ID NO: 4 | Oligonukleotidprimer GP1 5'-GATTGTCTCTAGATGTAGGTGTGTTT-3' |
| | |
| 5. SEQ ID NO: 5 | Oligonukleotidprimer GP2 5'-CATGGAAGC**CAT**ggTTGAATTTCT-3' |
| | |
| 6. SEQ ID NO: 6 | Oligonukleotidprimer GPSP 5'-TTCCTGATCCaTGgCTTTCTGTTTCGC-3' |
| | |
| 7. SEQ ID NO: 7 | Nukleinsäuresequenz kodierend für das Transitpeptid der plastidäre 1,4-α-D-Glukanphosphat-α-D-glucosyltransferase aus Vicia faba |
| | |
| 8. SEQ ID NO: 8 | Aminosäuresequenz kodierend für das Transitpeptid der plastidäre 1,4-α-D-Glukanphosphat-α-D-glucosyltransferase aus Vicia faba |

### Beschreibung der Abbildungen

1. Fig. 1: Analyse von Knollen mit PTGPGUS-kan transformierter Kartoffelpflanzen. Wiedergegeben ist die Färbung X-Gluc inkubierter Scheiben von Knollen zweier unabhängiger Linien (A und B; die Dunkelfärbung entspricht der Blaufärbung).
2. Fig. 2: Analyse von Früchten mit PTGPGUS-kan transformierten Tomatenpflanzen. Gezeigt sind Früchte einer transgenen Linie (gp7) im Vergleich zu Wildtyp-Früchten. Die Dunkelfärbung bei den transgene Früchten entspricht der GUS-Blaufärbung. Die Dunkelfärbung bei den Wildtyp-Früchten entspricht der natürlichen Rotfärbung der Früchte; eine Blaufärbung ist hier nicht zu erkennen. 1: grüne, unreife Frucht; 2: orangefarbene Frucht; 3: rote, reife Frucht; 4: überreife Frucht. Man erkennt, dass die Blaufärbung mit dem Reifegrad der Frucht abnimmt.
3. Fig. 3: Analyse von Früchten mit PTUSPGUS-kan transformierter Tomatenpflanzen. A: unreife Frucht; B: reife Frucht. Der USP-Promotor zeigt im Unterschied zu den erfindungsgemäßen Promotoren nur eine Aktivität in den Samenkörnern.
4. Fig. 4: Stärkefärbung von Tomatenscheiben unterschiedlicher Entwicklungsstadien. Obere Reihe (A) ungefärbt; untere Reihe (B) Stärkefärbung mit Lugol'scher Lösung. Man erkennt, dass die Färbung mit dem Reifegrad der Frucht abnimmt.

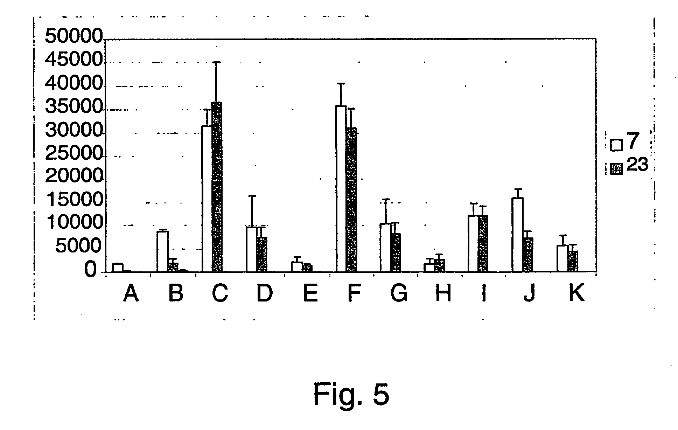

5. Fig. 5: Analyse von Früchten mit PTGPGUS-kan transformierten Tomatenpflanzen. Gezeigt sind die GUS-Expressionsaktivitäten in Geweben zweier transgener Linie (7 und 23). Wildtyp-Früchte ergaben jeweils nur eine Hintergrundniveau. A: Blatt; B: Wurzel; C: Schale, grün; D; Schale, orange; E: Schale, rot; F: Fleisch, grün; G: Fleisch, orange; H: Fleisch, rot; I: Samen, grün; J: Samen, orange; K: Samen, rot. Man erkennt eine hohe Aktivität v.a. im unreifen Fleisch und Schale der Tomate. (Y-Achse: GUS Aktivität gemessen in pM MU/min mg Protein; MU: Methylumbelliferon) Man erkennt, dass die GUS-Aktivität mit dem Reifegrad der Frucht abnimmt.

### Beispiele

### Allgemeine Methoden

Rekombinante DNA Techniken wurden entsprechend Maniatis et al., Molecular Cloning - A Laboratory Manual (Cold Spring Harbor Lab., Cold Spring Harbor, New York, 1982) durchgeführt. Die eingesetzten Enzyme wurden nach Vorschrift angewendet. Zur Klonierung wurden die Vektoren pUC18 (Yanisch-Perron C et al. (1985) Gene 33103-119), pBK-CMV (Stratagene) und pGUS1 (Pelemann J et al. (1989) Plant Cell 1:81-93) verwendet. Für die Pflanzentransformation wurden die Vektoren pGPTV-BAR und pGPTV-kan (Becker D et al. (1992) Plant Mol Biol 20:1195-1197) eingesetzt. Für die Transformation in E. coli wurde der Stamm DH5α (Hanahan D (1983) J Mol Biol 166;55-7-580) verwendet. Die Agrobakterienstämme EHA 105, GV3101[pMP90], C58Cl [pGV2260] und LBA4404 wurden nach An G (1987) Mol Gen Genet 207: 210-216 mittels Frost-Tau-Methode direkt transformiert.

### Beispiel 1: Klonierung des Promotors der Glukanphosphorylase von Vicia faba

Für die Isolierung des Promotors wurde genomische DNA von Vicia faba mit BlgII geschnitten und in das mit BamHI geschnittenen ZAP Express Vector System (#239212) von Stratagene ligiert und die Phagen ausplattiert. Mit einer Probe des cDNA Klons Pho1 (Genbank Acc.-No.: Z36880) wurde eine genomische *Vicia faba* DNA Bank (in pBKCMV (Stratagene) durchmustert und der genomische Klon pBKVfGP22 isoliert. Ausgehend von dem genomischen Klon pBKVfGP22 wurden
i) der 5' flankierende Bereich einschließlich der 5' untranslatierten Region und dem Startkodon ATG des Pho1 Gens und
ii) der 5' flankierende Bereich des Pho1 Gens einschließlich des putativen Transitpeptirles (Phol-TP) amplifiziert.

a) PCR Amplifikation des Pho1 Glukanphosphorylase Promotors
   Primer GP1 (SEQ ID NO: 4): 5'-GATTGTCTCTAGATGTAGGTGTGTTT-3'
   Primer GP2 (SEQ ID NO: 5): 5'-CATGGAAGC**CAT**ggTTGAATTTCT-3'
   Das Startkodon ATG (reverse complement) ist im Primer GP2 in Fettdruck dargestellt. Durch Austausch zweier T in g (Kleinbuchstaben) wurde der Erkennungsort für das Restriktionsenzym NcoI Ort direkt am Startkodon eingeführt.
   Reaktionsansatz:
   2 µl pBKVfGP22 (1:500)
   10 µl Ampli Taq Puffer
   0,5 µl Ampli Taq Polymerase
   2 µl dNTP (10 mM)
   2 µl GP1 (10 µM)
   2 µl GP2 (10 µM)
   81 µl 1 H₂O

   PCR Bedingungen:
   - 1 Zyklus:: 5 min. bei 96°C
   - 25 Zyklen:: 0,5 min. bei 48°C; 1 min. bei 72°C;
   0,5 min. bei 96°C
   - 1 Zyklus:: 0,5 min. bei 48°C; 10 min. bei 72°C
b) PCR Amplifikation des Glukan Phosphorylase Promotors einschließlich des Transitpeptides (Pho1-TP)
   Nach Buchner et al, (Planta 199:64-73, 1996) befindet sich eine Sequenz kodierend für ein plastidäres Transitpeptid am N-Terminus des Transkripts. Diese Sequenz zeigt keine signifikanten Homologien mit anderen chloroplastidären oder amyloplastidären Transitpeptiden. Nach der Regel von Gavel und von Heijne (FEBS Lett 261:455-458, 1990) korrespondiert die Sequenz mit dem Konsensus von Transitpeptiden. Das Transitpeptid hat danach eine Länge von 64 Aminosäuren. Der Primer GPSP wurde so gewählt, dass durch 10 zusätzliche Aminosäuren die Umgebung der Schnittstelle erhalten bleibt und so eine weitgehend sichere Prozessierung des Transitpeptide gewährleistet ist. Das Fragment wurden mit den Primers GP1 (s.o.; SEQ ID NO: 4) und GPSP (SEQ ID NO: 6) amplifiziert.
   Primer GPSP (SEQ ID NO: 6):
   5'-TTCCTGATCCaTGgCTTTCTGTTTCGC-3'
   Durch Austausch eines T in a und eines A in g (Kleinbuchstaben) wurde der Erkennungsort des Restriktionsenzyms NcoI eingeführt.
   Reaktionsansatz:
   2 µl pBKVfGP22 (1:500)
   10 µl Ampli Taq Puffer
   0,5 µl Ampli Taq Polymerase
   2 µl dNTP (10 mM)
   2 µl GP1 (10 µM)
   2 µl GPSP (10 µM)
   81 µl H₂O

   PCR Bedingungen:
   - 1 Zyklus:: 5 min. bei 96°C
   - 25 Zyklen:: 0,5 min. bei 48°C; 1 min. bei 72°C;
   0,5 min. bei 96°C
   - 1 Zyklus:: 0,5 min. bei 48°C; 10 min. bei 72°C

Die PCR Produkte wurden gereinigt und in den mit dem Restriktionsenzym SmaI geschnittenen Vektor pUC18 kloniert. Über Sequenzanalyse wurde die Sequenz der resultierenden Plasmide pGP und pGPSP2 verifiziert.

### Beispiel 2: Konstruktion der Expressionskassetten

### a) Konstruktion der Phol-Promotor-GUS Expressionskassette

Das Plasmid pGP wurde mit den Restriktionsenzymen PstI und Nco I gespalten. Für die Fusion mit dem GUS Gen wurde das ca. 1,4 kb große PstI/NcoI-Promotortragment in das ebenfalls mit PstI und NcoI geschnittenen Plasmid pGUS1 kloniert und die positiven Rekombinanten mittels einer HindIII Spaltung und Sequenzanalyse identifiziert. Das resultierende Plasmid wurde als pGPGUS bezeichnet.

### b) Konstruktion der Phol-TP-Promotor-GUS Expressionskassette

Das Plasmid pGPSP2 wurde mit den Restriktionsenzymen PstI und Nco I gespalten. Für die Fusion mit dem GUS Gen wurde das ca. 1,6 kb große PstI/NcoI-Promotorfragment in das ebenfalls mit PstI und NcoI geschnittenen Plasmid pGUS1 kloniert und die positiven Rekombinanten mittels einer HindIII Spaltung und Sequenzanalyse identifiziert. Das resultierende Plasmid wurde als pGPSPGUS bezeichnet.

### c) Klonierung der Phol-Promotor-GUS Expressionskassette in den Binärvektor pPTV-bar

Der Binärvektor pGPTV-bar wurde mit EcoRI und SmaI geschnitten, die Enden mit Klenow Enzym geglättet und religiert. Das resultierende Plasmid pPTV-bar diente als Binärvektor für die folgenden Konstruktionen. Das Plasmid pGPGUS wurde mit XbaI geschnitten und das den Glukanphosphorylase Promotor und das GUS Gen enthaltende Fragment in den Vektor pPTV-bar, gespalten mit XbaI, ligiert. Der resultierende Expressionsvektor (Plasmid) wurde als PTGPGUS bezeichnet.

### d) Klonierung der Pho1-TP-Promotor-GUS Expressionskassette in den Binärvektor pPTV-bar.

Das Plasmid pGPSPGUS wurde mit XbaI geschnitten und das den Glukanphosphorylase Promotor, das Transitpeptid und das GUS Gen enthaltende Fragment in den Vektor pPTV-bar, gespalten mit XbaI, ligiert. Der resultierende Expressionsvektor (Plasmid) wurde als PTGPSPGUS bezeichnet.

### e) Herstellung des Plasmids PTGPGUSKan

Für die Transformation in Kartoffel und Tomate wurde das Plasmid PTGPGUSKan hergestellt. Dazu wurde der Binärvektor pGPTV-kan mit EcoRI und SalI gespalten und das EcoRI/SalI geschnittene Fragment des Plasmids pGPGUS hinein kloniert.

### Beispiel 3; Transformation von Tabak, Raps, Arabidopsis, Kartoffel und Tomate

### a) Tabak

Zur Transformation von Tabakpflanzen (Nicotiana tabacum L. cv. Samsun NN) wurden 10 ml einer unter Selektion gewachsenen Übernachtkultur von Agrobakterium tumefaciens EHA105, transformiert mit den Expressionsvektoren PTGPGUS bzw. PTGPSPGUS, abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1 cm) in dieser Bakterienltssung gebadet. Anschließend wurden die Blattscheiben in Petrischalen auf MS-Medium (Murashige und Skoog (1962) Physiol Plant 15:473ff.) mit 2 % Saccharose und 0,8% Bacto-Agar ausgelegt. Nach zweitägiger Inkubation im Dunkeln bei 25°C wurden sie auf MS-Medium mit 100 mg/l Kanamycin, 500 mg/l Claforan, 1 mg/l Benzylaminopurin (BAP), 0,2 mg/l Naphthylessigsäure (NAA), 1,6 % Glukose und 0,8% Bacto-Agar übertragen und die Kultivierung (16 Stunden Licht / 8 Stunden Dunkelheit) fortgesetzt. Wachsende Sprosse wurden auf hormonfreies MS-Medium mit 2 % Saccharose, 250 mg/l Claforan und 0,8 % Bacto-Agar überführt.

### b) Raps

Die Transformation von Raps erfolgte mittels der Petiolentransformation nach Moloney et al. (Moloney MM et al. (1989) Plant Cell Reports 8:238-242). Der Agrobakteriumstamm EHA105, transformiert mit dem Expressionsvektor PTGPSPGUS, wurde für die Herstellung transgener Rapspflanzen eingesetzt.

### c) Arabidopsis

Die Transformation von *Arabidopsis* erfolgte mittels der "Floral Dip Methode" (Clough SJ und Bent AF (1998) Plant J 16:735-743). Der Agrobakteriumstamm EHA105, transformiert mit dem Expressionsvektor PTGPSPGUS, wurde für die Herstellung transgener Arabidopsispflanzen eingesetzt.

### d) Kartoffel

Zur Transformation von Kartoffel (Solanum tuberosum) wurden Blattscheiben von in vitro Planzen mit Agrobakterium tumefaciens C58Cl [pGV2260], transformiert mit dem Expressionsvektor PTGPGUSKan, in flüssigem Murashige Skoog Medium für 20 Minuten infiziert, und anschließend für 2 d im Dunkeln co-cultiviert. Nach der Co-cultur wurden die Explantate auf festes MS Medium, das anstelle von Saccharose 1,6 % Glucose enthält (MG) und mit 5 mg/l NAA, 0,1 mg/l BAP, 250 mg/l Timentin und 30 bis 40 mg/l Kanamycin ergänzt worden ist (KIM), bei 21°C in einem Licht/Dunkel-Rhythmus von 16h/8h kultiviert. Nach diese Kallusphase wurden die Explantate auf Sproßinduktionsmedium (SIM) gelegt. SIM war wie folgt zusammengesetzt: MG mit 2 mg/l Zeatinribosid, 0,02 mg/l NAA, 0,02 mg/l GA3, 250 mg/l Timentin, 30 bis 40 mg/l Kanamycin. Aller zwei Wochen wurden die Explantate auf frisches SIM transferiert. Die sich bildenden Sprosse wurden auf MS Medium mit 2 % Saccharose und 250 mg/l Timentin und 30 bis 40 mg/l Kanamycin bewurzelt.

### e). Tomate

Als Ausgangsexplantat für die Transformation dienen Kotyledonen sieben bis zehn Tage alter Keimlinge der Linie Microtom. Für die Keimung wird das Kulturmedium nach Murashige und Skoog (1962: Murashige and Skoog, 1962, Physiol. Plant 15, 473-) mit 2% Saccharose, pH 6,1 verwendet. Die Keimung findet bei 21°C bei wenig Licht (20 - 100 µE) statt. Nach sieben bis zehn Tagen werden die Kotyledonen quer geteilt und auf das Medium MSBN (MS, pH 6,1, 3 % Saccharose + 1mg/l BAP, 0,1 mg/l NAA) gelegt, das am Vortag mit suspensionskultivierten Tabakzellen beschickt wurde. Die Tabakzellen werden luftblasenfrei mit sterilem Filterpapier abgedeckt. Die Vorkultur der Explantate auf dem beschriebenen Medium erfolgt für drei bis fünf Tage. Anschließend werden die Explantate mit dem Agrobakterium tumefaciens Stamm LBA4404, der das binäre Plasmid mit dem zu transformierenden Gen trägt, wie folgt infiziert: Der Stamm, der Über Nacht in YEB Medium mit dem Antibiotikum für das Binärplasmid bei 28°C kultiviert bei worden ist, wird zentrifugiert. Das Bakterienpellet wird mit flüssigem MS Medium (3 % Saccharose, pH 6,1) resuspendiert und auf eine optische Dichte von 0,3 (bei 600 nm) eingestellt. Die vorkultivierten Explantate werden in die Suspension überführt und für 30 Minuten bei Zimmertemperatur unter leichtem Schütteln inkubiert. Anschließend werden die Explantate mit sterilem Filterpapier getrocknet und für die dreitägige Co-Kultur (21°C) auf ihr Vorkulturmedium zurück gelegt.

Nach der Co-kultur werden die Explantate auf MSZ2 Medium (MS pH 6,1 mit 3 % Saccharose, 2 mg/l Zeatin, 100 mg/l Kanamycin, 160 mg/l Timentin) transferiert und für die selektive Regeneration bei 21°C unter Schwachlicht Bedingungen (20 - 100 µE, Lichtrhythmus 16h/8h) aufbewahrt. Aller zwei bis drei Wochen erfolgt der Transfer der Explantate bis sich Sprosse bilden. Kleine Sprosse können vom Explantat abgetrennt werden und auf MS (pH 6,1 + 3 % Saccharose) 160 mg/l Timentin, 30 mg/l Kanamycin, 0,1 mg/l IAA bewurzelt werden. Bewurzelte Pflanzen werden ins Gewächshaus überführt.

### Beispiel 4: Isolierung genomischer DNA

Die genomische DNA transgener Tabak, Arabidopsis und Raps Pflanzen wurde mit Hilfe des DNA - Isolierungskit der Firma Macherey & Nagel isoliert. In einem ersten Schritt wurden die transgenen Linien über PCR mit genspezifischen Primern identifiziert. Die Integration der Fremd-DNA wurde mittels "Southernblot" - Analysen von 20 µg DNA nach geeigneter Restriktionsspaltung untersucht.

### Beispiel 5: Transiente Expressionsanalyse der Glukanphosphorylase Promotor-GUS Kassetten

Eine transiente Promotor Analyse wurde durchgeführt, um die Aktivität des Glukanphosphorylase-Promotor in verschiedenen Geweben und Pflanzen zu testen. Dazu wurde die Particel Gun "Biolistic PDS-1000System (BioRad Laboratories, Hercules, CA) mit einem Vakuum von 27mm Hg verwendet. Als Microcarrier wurden Goldpartikel mit einem Durchmesser von 1,0 µm benutzt, die nach der Vorschrift von BioRad mit Plasmid DNA beschichtet wurden. Dazu wurden 25µ einer Goldsuspension (50 mg/ml) mit 10 µl Qiagen gereinigte Plasmid DNA (1 µg/µl), 25 µl CaCl₂ (2,5 M) und 10 µl Spermidine (0,1 M) vermischt, kurz stehen gelassen und dann abzentrifugiert. Die beschichteten Goldpartikel wurden mit 70% und 100 % Ethanol gewaschen. Embryonen wurden mit 2000 PSI, Früchte von Tomaten und Knollen von Kartoffeln mit 1800 PSI beschossen. Die beschossenen Gewebe wurden 36 h in flüssigen Medium kultiviert und dann über Nacht bei 37°C mit einer X-Gluc Lösung behandelt. Die blauen Spots wurden ausgezählt.

Nach den Beschuß mit den Plasmiden pGPGUS und pGPSPGUS wurden in den Embryonen von Raps, Sonnenblume, Lein, Vicia faba und Sojabohne keine oder kaum sichtbare Spots gefunden. Das Beschießen von Scheiben der Kartoffelknolle und Fruchtgewebe von Tomate resultierte in deutlich mehr Spots, wobei keine wesentlichen Unterschiede zwischen den Plasmiden festgestellt werden konnten. Als Kontrolle wurde ein Plasmid verwendet, welches das GUS Gen unter der Kontrolle des USP (Unknown seed protein; Bäumlein et al. (1991) Mol Gen Genet 225:459-467) enthält. Obwohl der Promotor eine Expression spezifisch im Samen verursacht, zeigten sich eine Vielzahl von blauen Spots auch in anderen Sink-Geweben.

### Beispiel 6: Nachweis der gewebespezifischen Expression

Um die Eigenschaften des Promotors zu bestimmen, ist es erforderlich, den Promotor vor ein so genanntes Reportergen zu setzen, welches eine Bestimmung der Expressionsaktivität ermöglicht. Beispielsweise sei die bakterielle β-Glucuronidase genannt (Jefferson et al. (1987) EMBO J 6:3901-3907). Die β-Glucuronidase Aktivität kann *in planta* mittels eines chromogenen Substrates wie 5-Bromo-4-Chloro-3-Indolyl-β-D-Glucuronsäure (X-Gluc) im Rahmen einer Aktivitätsfärbung bestimmt werden. Für die Untersuchung der Gewebespezifität wird das pflanzliche Gewebe präpariert und gefärbt. Besonders vorteilhaft ist es Embryonen aus unreifen oder reifen Samen heraus zu präparieren und erst dann zu färben. Ebenso können ungeöffnete Blüten gefärbt werden, um eine Promotoraktivität in den Pollen nachzuweisen.

Ein zweiter Assay erlaubt eine quantitative Bestimmung der GUS Aktivität in dem untersuchten Gewebe. Für die quantitative Aktivitätsbestimmung wird als Substrat für die β-Glucuronidase MUG (4-Methyl-umbelliferyl-β-D-glucuronid) verwendet, das in MU (Methyl-umbelliferon) und Glucuronsäure gespalten wird.

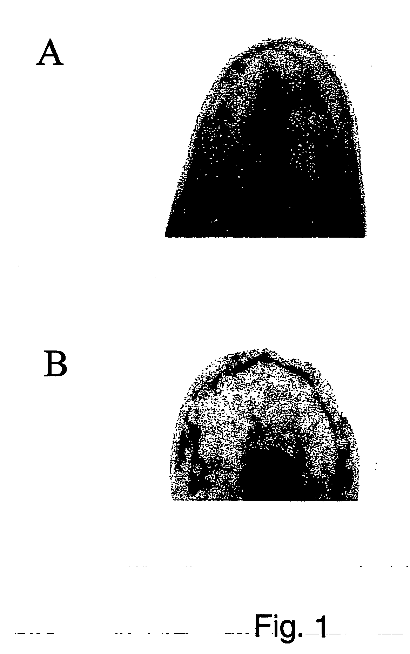

In den Knollen der analysierten Kartoffelpflanzen, transformiert mit dem Plasmid PTGPGUS-kan, konnte eine starke Expression des Glukanphosphorylase-Promotors nachgewiesen werden. Fig.1 zeigt die GUS Aktivität in Scheiben von Knollen zweier Linien. Abgesehen, von 2 Linien, welche eine kaum sichtbare Expression in den Blättern hatten, wurden keine Hinweise auf eine Expression in den Blättern gefunden. So zeigt sich auch hier die Korrelation der Expression in stärkehaltigen Geweben.

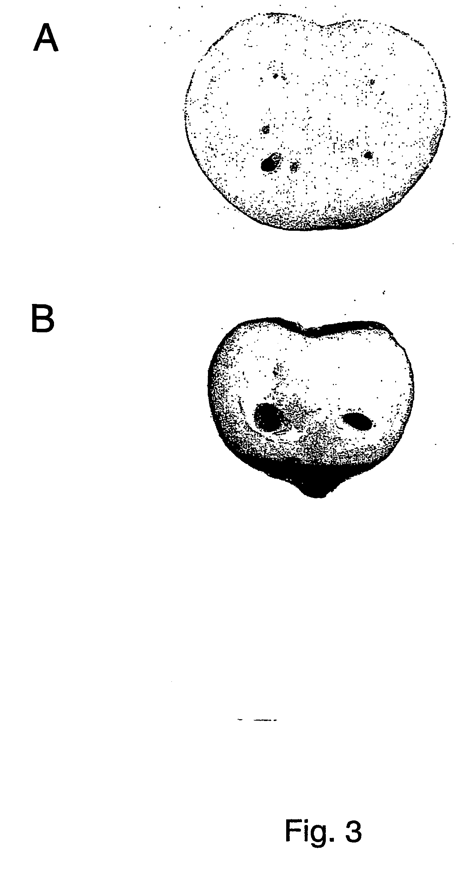

In den Früchten der transgenen Tomatenpflanzen,transformiert mit dem Plasmid PTGPGUS-kan, konnte eine starke Expression der Glucuronidase festgestellt werden. Fig. 2 zeigt die starke Expression während der Entwicklung der Tomatenfrucht. Im Gegensatz dazu zeigten die zum Vergleich hergestellten PTUSPGUSkan Pflanzen neben eine starken samenspezifische Aktivität (Fig.3) keine weiteren Aktivitäten dieses Promotors in den Früchten der transgenen Tomatenpflanzen.

In Tabak; Raps und Arabidopsis konnte keine wesentliche Aktivität des Glukanphosphorylase-Promotors detektiert werden.

### Beispiel 7: Korrelation von Stärkegehalt und Expressionsmuster des Glucan Phosphorylase Promotors in Tomatenfrüchten

Um die Korrelation zwischen stärkehaltigen Gewebe und Expression des Glukan Phosphorylase Promotors auf zu zeigen, wurden Tomatenscheiben mit Lugol'scher Lösung gefärbt. Eine starke Blaufärbung zeigt die Verteilung von Stärke an. Wie aus der Abb. 5 zu ersehen ist, wird gerade in den jungen Tomatenfrüchten viel Stärke detektiert. Das Verteilungsmuster stimmt auch mit dem GUS Expressionsmuster der transgenen Früchte überein.

## Patentansprüche

1. Verfahren zur gezielten, transgenen Expression von Nukleinsäuresequenzen in Kohlenhydrat-speichernden Sink-Geweben von Pflanzen enthaltend ein Kohlenhydrat-speicherndes Sink-Gewebe, wobei nachfolgende Schritte umfasst sind
I. Einbringen einer Expressionskassette in pflanzliche Zellen, wobei die Expressionskassette mindestens nachfolgende Elemente enthält
a) mindestens eine Promotorsequenz des Gens kodierend für die plastidäre 1,4-α-D-Glukanphosphat-α-D-glucosyltransferase aus Vicia faba, und
b) mindestens eine weitere Nukleinsäuresequenz,
wobei mindestens eine der besagten Promotorsequenzen und eine weitere Nukleinsäuresequenz funktionell miteinander verknüpft sind und die weitere Nukleinsäuresequenz in Bezug auf die Promotorsequenz heterolog ist, und
II. Auswahl von transgenen Zellen, die besagte Expressionskassette stabil in das Genom integriert enthalten, und
III. Regeneration von vollständigen Pflanzen aus besagten transgenen Zellen, wobei die Promotersequenz unter ansonsten unveränderten Bedingungen eine Transkription in mindestens einem Kohlenhydrat-speichernden, -synthetisierenden oder -metabolisierenden Sink-Gewebe vermittelt, die höher ist als in einem anderen Gewebe.

2. Verfahren nach Anspruch 1, wobei die Promotorsequenz des Gens kodierend für die plastidäre 1,4-α-D-Glukanphosphat-α-D-glucosyltransferase aus Vicia faba beschrieben ist durch eine Sequenz ausgewählt aus der Gruppe von Sequenzen bestehend aus
i) der Promotorsequenz gemäß SEQ ID NO: 1 und
ii) Promotorsequenzen, die eine Homologie von mindestens 80% über einen Sequenzabschnitt von mindestens 500 Basenpaaren zu der Sequenz gemäß SEQ ID NO: 1 aufweisen und die unter ansonsten unveränderten Bedingungen eine Transkription in mindestens einem Kohlenhydrat-speichernden, -synthetisierenden oder -metabolisierenden Sink-Gewebe vermittelt, die höher ist als in einem anderen Gewebe, und
iii) Fragmenten der Promotorsequenz gemäß i) mit einer Länge von mindestens 500 Basenpaaren und im wesentlichen der gleichen Promotoraktivität wie die Promotorsequenz gemäß SEQ ID NO: 1 und die unter ansonsten unveränderten Bedingungen eine Transkription in mindestens einem Kohlenhydrat-speichernden, -synthetisierenden oder -metabolisierenden Sink-Gewebe vermitteln, die höher ist als in einem anderen Gewebe.

3. Isolierte Nukleinsäuresequenz umfassend
i) die Promotorsequenz des Gens der plastidären 1,4-α-D-Glukanphosphat-α-D-glucosyltransferase aus Vicia faba gemäß SEQ ID NO: 1 oder
ii) Promotorsequenzen, die eine Homologie von mindestens 80% über einen Sequenzabschnitt von mindestens 500 Basenpaaren zu der Sequenz gemäß SEQ ID NO: 1 aufweisen und die unter ansonsten unveränderten Bedingungen eine Transkription in mindestens einem Kohlenhydrat-speichernden, -synthetisierenden oder -metabolisierenden Sink-Gewebe vermitteln, die höher ist als in einem anderen Gewebe, oder
iii)Fragmenten der Promotorsequenz gemäß i) mit einer Länge von mindestens 500 Basenpaaren und im wesentlichen der gleichen Promotoraktivität wie die Promotorsequenz gemäß SEQ ID NO: 1 und die unter ansonsten unveränderten Bedingungen eine Transkription in mindestens einem Kohlenhydrat-speichernden, -synthetisierenden oder -metabolisierenden Sink-Gewebe vermitteln, die höher ist als in einem anderen Gewebe.

4. Isolierte Nukleinsäuresequenz gemäß Anspruch 3, umfassend in 3'-Orientierung zu der Sequenz nach i) bis iii), eine Sequenz kodierend für ein Transitpeptid.

5. Isolierte Nukleinsauresequenz gemäß Anspruch 4, wobei das Transitpeptid durch eine Sequenz gemäß SEQ ID NO: 8 beschrieben wird.

6. Isolierte Nukleinsäuresequenz gemäß einem der Ansprüche 3 bis 5 beschrieben durch SEQ ID NO: 2 oder 3.

7. Expressionskassette zur Expression von Nukleinsäuren umfassend
a) mindestens eine Promotorsequenz des Gens kodierend für die plastidäre 1,4-α-D-Glukanphosphat-α-D-glucosyltransferase aus Vicia faba, und
b) mindestens eine weitere Nukleinsäuresequenz, und
wobei mindestens eine Promotorsequenz und eine weitere Nukleinsäuresequenz funktionell miteinander verknüpft sind und die weitere Nukleinsäuresequenz in Bezug auf die Promotorsequenz heterolog ist.

8. Expressionskassette nach Anspruch 7, wobei die Promotorsequenz des Gens kodierend für die plastidäre 1,4-α-D-Glukanphosphat-α-D-glucosyltransferase aus Vicia faba beschrieben ist durch eine Sequenz ausgewählt aus der Gruppe von Sequenzen bestehend aus
i) der Promotorsequenz gemäß SEQ ID NO: 1 und
ii) Promotorsequenzen, die eine Homologie von mindestens 80% über einen Sequenzabschnitt von mindestens 500 Basenpaaren zu der Sequenz gemäß SEQ ID NO: 1 aufweisen und die unter ansonsten unveränderten Bedingungen eine Transkription in mindestens einem Kohlenhydrat-speichernden, -synthetisierenden oder -metabolisierenden Sink-Gewebe vermitteln, die höher ist als in einem anderen Gewebe, und
iii) Fragmenten der Promotorsequenz gemäß i) mit einer Länge von mindestens 500 Basenpaaren und im wesentlichen der gleichen Promotoraktivität wie die Promotorsequenz gemäß SEQ ID NO: 1 und die unter ansonsten unveränderten Bedingungen eine Transkription in mindestens einem Kohlenhydrat-speichernden, -synthetisierenden oder -metabolisierenden Sink-Gewebe vermitteln, die höher ist als in einem anderen Gewebe.

9. Expressionskassette nach Anspruch 8, wobei die Promotorsequenz beschrieben ist durch eine Sequenz gemäß SEQ ID NO: 2 oder 3.

10. Expressionskassette nach einem der Ansprüche 7 bis 9, wobei die transgen zu exprimierende Nukleinsäuresequenz
a) die Expression eines von besagter Nukleinsäuresequenz kodierten Proteins, oder
b) die Expression eines von besagter Nukleinsäuresequenz kodierter sense-RNA, anti-sense-RNA oder doppelsträngigen RNA
ermöglicht.

11. Expressionsvektor enthaltend eine Nukleinsäuresequenz gemäß einem der Ansprüche 3 bis 6 oder eine Expressionskassette gemäß einem der Ansprüche 7 bis 10.

12. Pflanze enthaltend ein Kohlenhydrat-speicherndes Sink-Gewebe oder Mikroorganismus transformiert mit einer Expressionskassette gemäß einem der Ansprüche 7 bis 10 oder einem Expressionsvektor gemäß Anspruch 11.

13. Pflanze nach Anspruch 12 ausgewählt aus der Gruppe bestehend aus monocotyledonen oder dicotyledonen Pflanzen.

14. Pflanze nach einem der Ansprüche 12 oder 13 ausgewählt aus der Gruppe bestehend aus Tomate, Kartoffel, Aubergine, Sojabohne, Alfalfa, Erbse, Ackerbohne, Futterrübe, Zuckerrübe und Erdnuss.

15. Zellen, Zellkulturen, Teile, Organe, Gewebe oder transgenes Vermehrungsgut enthaltend eine Expressionskassette gemäß einem der Ansprüche 7 bis 10 oder einen Expressionsvektor gemäß Anspruch 11.

16. Verwendung einer isolierten Nukleinsäuresequenz gemäß einem der Ansprüche 3 bis 6 oder einer Expressionskassette gemäß einem der Ansprüche 7 bis 10 oder eines Expressionsvektors gemäß Anspruch 11 oder einer Pflanze gemäß einem der Anspruche 12 bis 14 oder von dieser abgeleitete Zellen, Zellkulturen, Teile, Organe, Gewebe oder transgenes Vermehrungsgut nach Anspruch 15 in Verfahren zur transgenen Expression von Nukleinsäuren oder Proteinen in Pflanzen enthaltend ein Kohlenhydrat-speicherndes Sink-Gewebe.

17. Verwendung einer isolierten Nukleinsäuresequenz gemäß einem der Ansprüche 3 bis 6 oder einer Expressionskassette gemäß einem der Ansprüche 7 bis 10 oder eines Expressionsvektors gemäß Anspruch. 11 oder einer Pflanze gemäß einem der Ansprüche 12 bis 14 oder von dieser abgeleitete Zellen, Zellkulturen, Teile, Organe, Gewebe oder transgenes Vermehrungsgut nach Anspruch 15 zur Herstellung von Nahrungs-, Futtermitteln, von Saatgut, Pharmazeutika oder Feinchemikalien mittels Pflanzen enthaltend ein Kohlenhydrat-speicherndes Sink-Gewebe.

18. Verfahren zur Herstellung von Nahrungs- oder Futtermitteln, Saatgut, Pharmazeutika oder Feinchemikalien, wobei man eine Pflanze gemäß einem der Ansprüche 12 bis 14 einsetzt und das gewünschte Nahrungs- oder Futtermittel, Saatgut, Pharmazeutikum oder die Feinchemikalie unter Verwendung des besagten Organismus herstellt und/oder isoliert.

## Claims

1. A method for the directed, transgenic expression of nucleic acid sequences in carbohydrate-storing sink tissues of plants comprising a carbohydrate-storing sink tissue, which comprises the following steps:
I. introducing, into plant cells, an expression cassette, where the expression cassette comprises at least the following elements:
a) at least one promoter sequence of the gene encoding the Vicia faba plastidic 1,4-a-D-glucan:phosphate α-D-glucosyltransferase, and
b) at least one further nucleic acid sequence,
where at least one of said promoter sequences and one further nucleic acid sequence are functionally linked with one another and the further nucleic acid sequence is heterologous with regard to the promoter sequence, and
II. selection of transgenic cells which comprise said expression cassette stably integrated into the genome, and
III. regeneration of intact plants from said transgenic cells, where the promoter sequence under otherwise unchanged conditions mediates, in at least one carbohydrate-storing, -synthesizing or -metabolizing sink tissue, a transcription which is higher than in another tissue.

2. The method according to claim 1, where the promoter sequence of the gene encoding the Vicia faba plastidic 1,4-a-D-glucan:phosphate α-D-glucosyltransferase is described by a sequence selected from the group of sequences consisting of
i) the promoter sequence of SEQ ID NO: 1 and
ii) promoter sequences which have at least 80% homology with the sequence of SEQ ID NO: 1 over a sequence segment of at least 500 base pairs and which under otherwise unchanged conditions mediate, in at least one carbohydrate-storing, -synthesizing or -metabolizing sink tissue, a transcription which is higher than in another tissue, and
iii) fragments of the promoter sequence of i) with a length of at least 500 base pairs and essentially the same promoter activity as the promoter sequence of SEQ ID NO: 1 and which under otherwise unchanged conditions mediate, in at least one carbohydrate-storing,
-synthesizing or -metabolizing sink tissue, a transcription which is higher than in another tissue.

3. An isolated nucleic acid sequence comprising
i) the promoter sequence of the gene of the Vicia faba plastidic 1,4-α-D-glucan:phosphate α-D-glucosyltransferase of SEQ ID NO: 1 or
ii) promoter sequences which have at least 80% homology with the sequence of SEQ ID NO: 1 over a sequence segment of at least 500 base pairs and which under otherwise unchanged conditions mediate, in at least one carbohydrate-storing, -synthesizing or -metabolizing sink tissue, a transcription which is higher than in another tissue, or
iii) fragments of the promoter sequence of i) with a length of at least 500 base pairs and essentially the same promoter activity as the promoter sequence of SEQ ID NO: 1 and which under otherwise unchanged conditions mediate, in at least one carbohydrate-storing, -synthesizing or -metabolizing sink tissue, a transcription which is higher than in another tissue.

4. The isolated nucleic acid sequence according to claim 3, comprising, in 3'-orientation relative to the sequence of i) to iii), a sequence encoding a transit peptide.

5. The isolated nucleic acid sequence according to claim 4, where the transit peptide is described by a sequence of SEQ ID NO: 8.

6. The isolated nucleic acid sequence according to any of claims 3 to 5, described by SEQ ID NO: 2 or 3.

7. An expression cassette for the expression of nucleic acids comprising
a) at least one promoter sequence of the gene encoding the Vicia faba plastidic 1,4-α-D-glucan:phosphate α-D-glucosyltransferase, and
b) at least one further nucleic acid sequence, and
where at least one promoter sequence and one further nucleic acid sequence are functionally linked with one another and the further nucleic acid sequence is heterologous with regard to the promoter sequence.

8. The expression cassette according to claim 7, where the promoter sequence of the gene encoding the Vicia faba plastidic 1,4-α-D-glucan:phosphate α-D-glucosyltransferase is described by a sequence selected from the group of sequences consisting of
i) the promoter sequence of SEQ ID NO: 1 and
ii) promoter sequences which have at least 80% homology with the sequence of SEQ ID NO: 1 over a sequence segment of at least 500 base pairs and which under otherwise unchanged conditions mediate, in at least one carbohydrate-storing, -synthesizing or -metabolizing sink tissue, a transcription which is higher than in another tissue, and
iii) fragments of the promoter sequence of i) with a length of at least 500 base pairs and essentially the same promoter activity as the promoter sequence of SEQ ID NO: 1 and which under otherwise unchanged conditions mediate, in at least one carbohydrate-storing, -synthesizing or -metabolizing sink tissue, a transcription which is higher than in another tissue.

9. The expression cassette according to claim 8, where the promoter sequence is described by a sequence of SEQ ID NO: 2 or 3.

10. The expression cassette according to any of claims 7 to 9, where the nucleic acid sequence to be expressed transgenically makes possible
a) the expression of a protein encoded by said nucleic acid sequence, or
b) the expression of a sense RNA, antisense RNA or double-stranded RNA encoded by said nucleic acid sequence.

11. An expression vector comprising a nucleic acid sequence according to any of claims 3 to 6 or an expression cassette according to any of claims 7 to 10.

12. A plant comprising a carbohydrate-storing sink tissue or microorganism transformed with an expression cassette according to any of claims 7 to 10 or an expression vector according to claim 11.

13. The plant according to claim 12, selected from the group consisting of monocotyledonous or dicotyledonous plants.

14. The plant according to one of claims 12 or 13, selected from the group consisting of tomato, potato, aubergine, soybean, alfalfa, pea, field bean, fodder beet, sugar beet and peanut.

15. A cell, cell culture, part, organ, tissue or transgenic propagation material comprising an expression cassette according to any of claims 7 to 10 or an expression vector according to claim 11.

16. The use of an isolated nucleic acid sequence according to any of claims 3 to 6 or of an expression cassette according to any of claims 7 to 10 or of an expression vector according to claim 11 or of a plant according to any of claims 12 to 14 or of cells, cell cultures, parts, organs, tissues or transgenic propagation material derived therefrom according to claim 15 in methods for the transgenic expression of nucleic acids or proteins in plants comprising a carbohydrate-storing sink tissue.

17. The use of an isolated nucleic acid sequence according to any of claims 3 to 6 or of an expression cassette according to any of claims 7 to 10 or of an expression vector according to claim 11 or of a plant according to any of claims 12 to 14 or of cells, cell cultures, parts, organs, tissues or transgenic propagation material derived therefrom according to claim 15 for the production of foodstuffs, feedstuffs, seed, pharmaceuticals or fine chemicals by means of plants comprising a carbohydrate-storing sink tissue.

18. A method for the production of foodstuffs or feedstuffs, seed, pharmaceuticals or fine chemicals, in which a plant according to one of claims 12 to 14 is used and the desired foodstuff or feedstuff, seed, pharmaceutical or the fine chemical is produced and/or isolated using said organism.

## Revendications

1. Procédé pour l'expression transgénique ciblée de séquences d'acide nucléique dans des tissus-puits, emmagasinant les glucides, de plantes contenant un tissu-puits emmagasinant les glucides, comprenant les étapes suivantes
I. introduction d'une cassette d'expression dans des cellules végétales, la cassette d'expression contenant au moins les éléments suivants
a) au moins une séquence de promoteur du gène codant pour la 1,4-α-D-glucane-phosphate-α-D-glucosyltransférase plastidique de *Vicia faba,* et
b) au moins une autre séquence d'acide nucléique,
au moins l'une desdites séquences de promoteurs et une autre séquence d'acide nucléique étant fonctionnellement liées l'une à l'autre et l'autre séquence d'acide nucléique étant hétérologue eu égard à la séquence de promoteur, et
II. choix de cellules transgéniques qui contiennent ladite cassette d'expression intégrée de façon stable dans le génome, et
III. régénération de plantes complètes à partir desdites cellules transgéniques, la séquence de promoteur induisant dans des conditions par ailleurs inchangées une transcription, dans au moins un tissu-puits emmagasinant, synthétisant ou métabolisant les glucides, qui est plus élevée que dans un autre tissu.

2. Procédé selon la revendication 1, dans lequel la séquence de promoteur du gène codant pour la 1,4-α-D-glucanephosphate-α-D-glucosyltransférase plastidique de *Vicia faba* est décrite par une séquence choisie dans le groupe de séquences constitué par
i) la séquence de promoteur selon SEQ ID n° 1 et
ii) des séquences de promoteurs qui présentent une homologie d'au moins 80 % sur un segment de séquence d'au moins 500 paires de bases avec la séquence selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu, et
iii) des fragments de la séquence de promoteur selon i) ayant une longueur d'au moins 500 paires de bases et essentiellement la même activité de promoteur que la séquence de promoteur selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu.

3. Séquence d'acide nucléique isolée, comprenant
i) la séquence de promoteur du gène codant pour la 1,4-α-D-glucanephosphate-α-D-glucosyl-transférase plastidique de *Vicia faba* selon SEQ ID n° 1 ou
ii) des séquences de promoteurs qui présentent une homologie d'au moins 80 % sur un segment de séquence d'au moins 500 paires de bases avec la séquence selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu, ou
iii) des fragments de la séquence de promoteur selon i) ayant une longueur d'au moins 500 paires de bases et essentiellement la même activité de promoteur que la séquence de promoteur selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu.

4. Séquence d'acide nucléique isolée, selon la revendication 3, comprenant en orientation 3' par rapport à la séquence selon i) à iii), une séquence codant pour un peptide de transit.

5. Séquence d'acide nucléique isolée, selon la revendication 4, le peptide de transit étant décrit par une séquence selon SEQ ID n° 8.

6. Séquence d'acide nucléique isolée, selon l'une quelconque des revendications 3 à 5, décrite par SEQ ID n° 2 ou 3.

7. Cassette d'expression pour l'expression d'acides nucléiques, comprenant
a) au moins une séquence de promoteur du gène codant pour la 1,4-α-D-glucanephosphate-α-D-glucosyltransférase plastidique de *Vicia faba,* et
b) au moins une autres séquence d'acide nucléique, et
au moins une séquence de promoteur et une autres séquence d'acide nucléique étant fonctionnellement liées l'une à l'autre et l'autre séquence d'acide nucléique étant hétérologue vis-à-vis de la séquence de promoteur.

8. Cassette d'expression selon la revendication 7, dans laquelle la séquence de promoteur du gène codant pour la 1,4-α-D-glucanephosphate-α-D-glucosyltransférase plastidique de *Vicia faba* est décrite par une séquence choisie dans le groupe des séquences constitué par
i) la séquence de promoteur selon SEQ ID n° 1 et
ii) des séquences de promoteurs qui présentent une homologie d'au moins 80 % sur un segment de séquence d'au moins 500 paires de bases avec la séquence selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu, et
iii) des fragments de la séquence de promoteur selon i) ayant une longueur d'au moins 500 paires de bases et essentiellement la même activité de promoteur que la séquence de promoteur selon SEQ ID n° 1 et qui induisent dans des conditions par ailleurs inchangées une transcription, dans au moins un tissu-puits emmagasinant, synthétisant ou métabolisant les glucides, qui est plus élevée que dans un autre tissu.

9. Cassette d'expression selon la revendication 8, dans laquelle la séquence de promoteur est décrite par une séquence selon SEQ ID n° 2 ou 3.

10. Cassette d'expression selon l'une quelconque des revendications 7 à 9, dans laquelle la séquence d'acide nucléique à exprimer de façon transgénique permet
a) l'expression d'une protéine codée par ladite séquence d'acide nucléique, ou
b) l'expression d'un ARN sens, ARN antisens ou ARN double brin, codé par ladite séquence d'acide nucléique.

11. Vecteur d'expression contenant une séquence d'acide nucléique selon l'une quelconque des revendications 3 à 6 ou une cassette d'expression selon l'une quelconque des revendications 7 à 10.

12. Plante contenant un tissu-puits emmagasinant les glucides ou micro-organisme transformé par une cassette d'expression selon l'une quelconque des revendications 7 à 10 ou un vecteur d'expression selon la revendication 11.

13. Plante selon la revendication 12, choisie dans le groupe constitué par les plantes monocotylédones et les plantes dicotylédones.

14. Plante selon la revendication 12 ou 13, choisie dans le groupe constitué par la tomate, la pomme de terre, l'aubergine, le soja, la luzerne, le pois, la féverole, la betterave fourragère, 1 betterave à sucre et l'arachide.

15. Cellules, cultures de cellules, parties, organes, tissu ou matériel de multiplication transgénique, contenant une cassette d'expression selon l'une quelconque des revendications 7 à 10 ou un vecteur d'expression selon la revendication 11.

16. Utilisation d'une séquence d'acide nucléique isolée, selon l'une quelconque des revendications 3 à 6, ou d'une cassette d'expression selon l'une quelconque des revendications 7 à 10 ou d'un vecteur d'expression selon la revendication 11 ou d'une plante selon l'une quelconque des revendications 12 à 14 ou de cellules, cultures de cellules, parties, organes, tissus ou matériel de multiplication transgénique, dérivés de ceux-ci, selon la revendication 15, dans des procédés pour l'expression transgénique d'acides nucléiques ou de protéines dans des plantes contenant un tissu-puits emmagasinant les glucides.

17. Utilisation d'une séquence d'acide nucléique isolée, selon l'une quelconque des revendications 3 à 6, ou d'une cassette d'expression selon l'une quelconque des revendications 7 à 10 ou d'un vecteur d'expression selon la revendication 11 ou d'une plante selon l'une quelconque des revendications 12 à 14 ou de cellules, cultures de cellules, parties, organes, tissu ou matériel de multiplication transgénique, dérivés de ceux-ci, selon la revendication 15, pour la production de produits alimentaires, d'aliments pour animaux, de semences, de produits pharmaceutiques ou de produits de chimie fine au moyen de plantes contenant un tissu-puits emmagasinant les glucides.

18. Procédé pour la production de produits alimentaires, d'aliments pour animaux, de semences, de produits pharmaceutiques ou de produits de chimie fine, dans lequel on utilise une plante selon l'une quelconque des revendication 12 à 14 et on produit et/ou isole le produit alimentaire, l'aliment pour animaux, la semence, le produit pharmaceutique ou le produit de chimie fine recherchés, en utilisant ledit organisme.
